(19) Europäisches Patentamt
European Patent Office
Office européen des brevets

(11) EP 2 319 922 B1

(12) **EUROPEAN PATENT SPECIFICATION**

(45) Date of publication and mention of the grant of the patent:
**14.10.2015 Bulletin 2015/42**

(21) Application number: **08791869.4**

(22) Date of filing: **30.07.2008**

(51) Int Cl.:
***C12N 15/09*** *(2006.01)* ***C12Q 1/68*** *(2006.01)*

(86) International application number:
**PCT/JP2008/063638**

(87) International publication number:
**WO 2010/013317 (04.02.2010 Gazette 2010/05)**

(54) **NUCLEIC ACID PROBE SET AND METHOD OF USING THE SAME**

NUKLEINSÄURESONDENSATZ UND VERFAHREN ZUR VERWENDUNG DAVON

ENSEMBLE DE SONDES D'ACIDES NUCLÉIQUES ET LEUR PROCÉDÉ D'UTILISATION

(84) Designated Contracting States:
**AT BE BG CH CY CZ DE DK EE ES FI FR GB GR HR HU IE IS IT LI LT LU LV MC MT NL NO PL PT RO SE SI SK TR**

(43) Date of publication of application:
**11.05.2011 Bulletin 2011/19**

(73) Proprietor: **Nippon Steel Kankyo Engineering Co., Ltd.**
**Chiyoda-ku**
**Tokyo 101-0031 (JP)**

(72) Inventors:
- **ICHIKAWA, Kohei**
  **Tokyo 101-0031 (JP)**
- **NAKAMURA, Kazunori**
  **Tokyo 101-0031 (JP)**
- **KURATA, Shinya**
  **Tokyo 101-0031 (JP)**

(74) Representative: **Hartz, Nikolai**
**Wächtershäuser & Hartz**
**Patentanwaltspartnerschaft mbB**
**Weinstrasse 8**
**80333 München (DE)**

(56) References cited:
**WO-A1-02/08414** **WO-A1-2005/059548**
**WO-A2-02/053778** **WO-A2-2008/021446**
**JP-A- 2001 286 300** **JP-A- 2008 182 974**
**JP-A- 2008 182 974**

- **TANI ET AL: "Calibration-curve-free quantitative PCR: A quantitative method for specific nucleic acid sequences without using calibration curves", ANALYTICAL BIOCHEMISTRY, ACADEMIC PRESS INC, NEW YORK, vol. 369, no. 1, 28 August 2007 (2007-08-28), pages 105-111, XP022217971, ISSN: 0003-2697, DOI: 10.1016/J.AB.2007.06.047**
- **TANI HIDENORI ET AL: "Quantitative method for specific nucleic acid sequences using competitive polymerase chain reaction with an alternately binding probe", ANALYTICAL CHEMISTRY, AMERICAN CHEMICAL SOCIETY, US, vol. 79, no. 3, 1 February 2007 (2007-02-01), pages 974-979, XP002508388, ISSN: 0003-2700, DOI: 10.1021/AC0615060**
- **KAZUNORI NAKAMURA: "Idenshi Teiryo Kaiseki Bunya no Honkaku Kenkyu Keiko Shoko Gensho o Riyo shita Idenshi Teiryo Kaiseki Gijutsu", AIST TODAY, SANGYOU GIJUTSU SOUGOU KENKYUUJO SEIKA FUKYUU BUMON, TSUKUBA, JP, vol. 8, no. 2, 1 February 2008 (2008-02-01), pages 18-19, XP008144198, ISSN: 1346-5805**
- **NODA ET AL: "Estimation of single-nucleotide polymorphism allele frequency by alternately binding probe competitive polymerase chain reaction", ANALYTICA CHIMICA ACTA, ELSEVIER, AMSTERDAM, NL, vol. 608, no. 2, 23 December 2007 (2007-12-23), pages 211-216, XP022428147, ISSN: 0003-2670, DOI: 10.1016/J.ACA.2007.12.015**

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

EP 2 319 922 B1

- **TANI H ET AL: "Universal quenching probe system: flexible, specific, and cost-effective real-time polymerase chain reaction method", ANALYTICAL CHEMISTRY, AMERICAN CHEMICAL SOCIETY, US, vol. 81, no. 14, 15 July 2009 (2009-07-15) , pages 5678-5685, XP008136132, ISSN: 0003-2700, DOI: 10.1021/AC900414U [retrieved on 2009-06-16]**
- **TANI HIDENORI ET AL.: 'Calibration-curve-free quantitative PCR: a quantitative method for specific nucleic acid sequences without using calibration curves.' ANALYTICAL BIOCHEMISTRY vol. 369, no. 1, 2007, pages 105 - 111, XP022217971**
- **NODA NAOHIRO ET AL.: 'Estimation of single-nucleotide polymorphism allele frequency by alternately binding probe competitive polymerase chain reaction.' ANALYTICA CHIMICA ACTA vol. 608, no. 2, February 2008, pages 211 - 216, XP022428147**
- **KAZUNORI NAKAMURA: 'Idenshi Teiryo Kaiseki Bunya no Honkaku Kenkyu Keiko Shoko Gensho o Riyo shita Idenshi Teiryo Kaiseki Gijutsu' AIST TODAY vol. 8, no. 2, 01 February 2008, pages 18 - 19, XP008144198**

SEQUENCE LISTING

<110> Nippon Steel Kankyo Engineering Co.,Ltd.
J-Bio 21 Corporation
<120> Nucleic acid probe set and use thereof
<130> PCT-71-JBEN
<160> 12

<210> 1
<211> 21
<212> DNA
<213> Homo sapiens
<400> 1
tgaccacagt ttgatcgagt g 21

<210> 2
<211> 39
<212> DNA
<213> Artificial Sequence
<220>

<221> modified_base
<222> 39
<223> phosphorylated
<400> 2
tctcggagtc cgggcgatca ctcgatcaaa ctgtggtca 39

<210> 3
<211> 19
<212> DNA
<213> Artificial Sequence <220>
<221> modified_base
<222> 1
<223> labeled with BODIPY-FL
<220>
<221> modified_base
<222> 19
<223> phosphorylated
<400> 3
catcgcccgg actccgaga 19

<210> 4
<211> 20
<212> DNA
<213> Artificial Sequence
<220>
<223> forward primer
<400> 4
agtggtggct aatgagagaa 20

<210> 5
<211> 17
<212> DNA
<213> Artificial Sequence
<220>
<223> reverse primer
<400> 5
aaggagtggc agcaagt 17

<210> 6
<211> 21
<212> DNA
<213> Artificial Sequence
<220>
<223> mutant of sequense No.1
<400> 6
tgaccacagt ttgatcaagt g 21

<210> 7
<211> 25
<212> DNA
<213> Nitrosomonas europaea
<400> 7
gtcaccagcc agttgcgtgt cagat 25

<210> 8
<211> 55
<212> DNA
<213> Artificial Sequence

<220>
<223> binding probe
<400> 8
attcgggcgg gcttaatctg acacgcaact ggctggtgac tttggggggg ggttt 55

<210> 9
<211> 15
<212> DNA
<213> Artificial Sequence
<220>
<221> modified_base
<222> 1
<223> labeled with BODIPY-F1,
<220>
<221> modified_base
<222> 15
<223> phosphorylated
<400> 9
taagcccgcc cgaat 15

<210> 10
<211> 15
<212> DNA
<213> Artificial Sequence
<220>
<221> modified_base
<222> 15
<223> TAMRA
<400> 10
aaaccccccc ccaaa 15

<210> 11
<211> 18
<212> DNA
<213> Artificial Sequence
<220>
<223> forward primer
<400> 11
gghgactggg ayttctgg 18

<210> 12
<211> 19
<212> DNA
<213> Artificial Sequence
<220>
<223> reverse primer
<400> 12
cctckgsaaa gccttcttc 19

## Description

### Technical Field

This relates to the field of genetic engineering, and more specifically, to a nucleic acid probe set useful for analyzing a nucleic acid and a method for using the same.

### Background Art

In detection methods of target nucleic acids, single-stranded nucleic acid probes that specifically hybridize to the target nucleic acids are widely used. As one of these detection methods of target nucleic acids, said detection methods making use of the single-stranded nucleic acid probes, there is the Q-probe (Quenching Probe) method. According to this Q-probe method, such a nucleic acid probe is labeled with a fluorescent substance. When this nucleic acid probe hybridizes to a target nucleic acid, fluorescence is quenched under the action of guanine in the target nucleic acid. Upon dissociation of the nucleic acid probe from the target nucleic acid, fluorescence is re-emitted. The Q-probe method utilizes this phenomenon. The Q-probe method has excellent advantages in that the structure of the probe is simple, no trial-and-error approach is needed for the designing of the probe, and highly-accurate results can be obtained (see, for example, Patent Document 1 and Patent Document 2).

Patent Document 1: JP-B-3437816
Patent Document 2: JP-A-2002-119291

### Disclosure of the Invention

### Problem to Be Solved by the Invention

As such a conventional, single-stranded nucleic acid probe, however, a fluorescently-labeled nucleic acid probe having a different base sequence has to be prepared specifically for every target nucleic acid to be detected. Such a fluorescently-labeled nucleic acid probe is accompanied by problems that it is relatively costly and its synthesis requires a long time, and therefore, involves problems that an experiment making use of it is costly and requires a lot of time in preparation.
Objects of the present invention are, therefore, to provide a nucleic acid probe, which can solve the above-mentioned problems of the conventional technology and can be prepared in a short time and economically, and also a method for using the same.

### Means for Solving the Problem

The above-described objects can be achieved by the present invention as described in claims 1, 2 and 3.
Described specifically, the present invention provides a nucleic acid probe set comprising (A) one or two fluorescent probe or probes, each of which is formed of an oligonucleotide including (a) a nucleotide labeled with (d) a fluorescent substance, and (B) a binding probe formed of an oligonucleotide having (b1) one or two fluorescent probe binding region or regions, to which the fluorescent probe or probes (A) can hybridize, and (b2) a target nucleic acid binding region, which can hybridize to a target nucleic acid (C), wherein the nucleic acid probe set is designed such that, when the fluorescent probe or probes (A) hybridizes or hybridize to the fluorescent probe binding region or regions (b1) and the target nucleic acid (C) hybridizes to the target nucleic acid binding region (b2), guanine in a nucleic acid, said nucleic acid including the target nucleic acid (C), and the fluorescent substance (d) labeled on the nucleotide (a) interact with each other to change a fluorescent character of the fluorescent substance (d).
In the above-described nucleic acid probe set according to the present invention, it is preferred that the fluorescent substance (d) is any one selected from the group consisting of fluorescein, fluorescein-4-isothiocyanate, tetrachlorofluorescein, hexachlorofluorescein, tetrabromosulfonefluorescein, EDANS, 6-JOE, Alexa Fluor 488, Alexa Fluor 532, Pacific Blue, rhodamine 6G, carboxyrhodamine 6G, tetramethylrhodamine, carboxytetramethylrhodamine and BODIPY-FL; that the one or two fluorescent probe or probes (A) each comprise an oligonucleotide labeled at a 5'-terminal nucleotide or 3'-terminal nucleotide thereof with the fluorescent substance (d); that the one or two fluorescent probe or probes (A) are each 5 to 50 bases long; and that the target nucleic acid binding region (b2) is 5 to 60 bases long.
The present invention also provides a method for detecting a target nucleic acid, which comprises the following steps (1) to (4):

(1) hybridizing the nucleic acid probe set according to the present invention and the target nucleic acid (C) with each other,
(2) then measuring the fluorescence intensity of a hybridized complex of the nucleic acid probe set and target nucleic acid (C),
(3) conducting the steps (1) and (2) by changing a ratio of the nucleic acid probe set to the target nucleic acid, and
(4) comparing the fluorescence intensities obtained from the steps (2) and (3); and also
a real-time PCR method, which comprises using the nucleic acid probe according to the present invention.

The present invention further provides a method for analyzing a nucleic acid for a base sequence polymorphism, which comprises the following steps (1) to (4) :

(1) hybridizing the nucleic acid probe set according to the present invention and a target nucleic acid (C) with each other,
(2) then measuring a temperature dependence of fluorescence intensity with respect to a hybridized complex of the nucleic acid probe set and target nucleic acid (C),
(3) conducting the steps (1) and (2) by using another nucleic acid in place of the target nucleic acid, and
(4) comparing the temperature dependences of fluorescence intensity as obtained from the steps (2) and (3).

The present invention still further provides a method, which comprises conducting a melting curve analysis on a complex of the nucleic acid probe set according to the present invention and a target nucleic acid (C).

The present invention even still further provides the nucleic acid probe set according to the present invention, wherein the fluorescent probe (A) consists of two fluorescent probes, which have different base sequences and are labeled with different fluorescent substances, respectively, and the binding probe (B) has two fluorescent probe binding regions having different base sequences, and an ABC-PCR method, which comprises using the nucleic acid probe set.

**Advantageous Effects of the Invention**

As nucleic acid probes for use in various analysis methods of nucleic acids, it has conventionally been needed to prepare, specifically for every target nucleic acid to be analyzed, one labeled with a costly fluorescent substance. According to the present invention, however, it is unnecessary to prepare a nucleic acid probe, which is labeled with a costly fluorescent substance, specifically for every target nucleic acid. Compared with such conventional nucleic acid probes, nucleic acid probes can, therefore, be provided economically and in a short time.

**Best Modes for Carrying out the Invention**

Best modes for carrying out the present invention will next be described with reference to drawings. It is to be noted that in the present invention, the hybridized complex of the fluorescent probe (A) and binding probe (B) may be called "the nucleic acid probe complex".

Further, the term "nucleotide" as used herein means a monomer of a deoxyribonucleotide or ribonucleotide, and the term "oligonucleotide" as used herein means an oligomer formed from a nucleotide. This oligomer may be formed from only a deoxyribonucleotide or a ribonucleotide, or may contain both of them.

The term "target nucleic acid" as used herein means the region of a base sequence that specifically hybridizes with the target nucleic acid binding region (b2) in the binding probe (B) which constitutes the nucleic acid probe set according to the present invention, and shall be construed that it may include a portion or portions of one or more of various nucleic acids or genes in some instances. The target nucleic acid can be of any type, and DNA, RNA, LNA, PNA artificially-modified nucleic acids, and the like can be mentioned.

Examples of the nucleic acid probe set according to the present invention are shown in FIG. 1, FIG. 2 and FIG. 8. In these figures, there are shown fluorescent probes (A) and binding probes (B). The nucleic acid probe set according to the present invention comprises one or two of such fluorescent probes (A) and one of such binding probe (B). Sign (C) indicates one example of the target nucleic acid.

FIG. 1 is a schematic diagram showing a nucleic acid probe complex in the present invention, which has one fluorescent probe (A), one fluorescent probe binding region (b1) and one target nucleic acid binding region (b2) and which is before hybridization with the target nucleic acid (C). FIG. 2 illustrates a state in which the target nucleic acid (C) and the nucleic acid probe complex in the present invention, both shown in FIG. 1, have hybridized with each other. FIG. 8 is a schematic diagram illustrating another nucleic acid probe complex in the present invention, which has two fluorescent probes (A) and two fluorescent probe binding regions (b1) and which is before hybridization with the target nucleic acid (C).

The one or two fluorescent probe or probes (A), which constitutes or constitute the nucleic acid probe set according to the present invention and may hereinafter be called simply "the fluorescent probe (A)", are each the oligonucleotide including the nucleotide (a) labeled with the fluorescent substance (d). No particular limitation is imposed on the base sequence of the fluorescent probe (A) insofar as it can hybridize with the fluorescent probe binding region (b1) in the binding probe (B). The same fluorescent probe can, therefore, be always used irrespective of the type of a target nucleic acid to be detected or analyzed. As the fluorescent probe (A) that constitutes the nucleic acid probe set according to the present invention is not required to have a base sequence corresponding to a specific target nucleic acid as mentioned above, the use of the nucleic acid probe set according to the present invention for the analysis of a target nucleic acid has an advantage in that it is no longer needed to prepare a fluorescent probe, which has a costly fluorescent substance, specifically for the target nucleic acid to be detected or analyzed.

On the other hand, the binding probe (B) that also constitutes the nucleic acid probe set according to the present invention is the oligonucleotide having the one or two fluorescent probe binding region or regions (b1) and the target nucleic acid binding region (b2). The one or two fluorescent probe binding region or regions (b1) hybridizes or hybridize with the fluorescent probe or probes (A), and may hereinafter be called simply "the fluorescent probe binging region (b1)". The target nucleic acid binding region (b2) hybridizes with the target nucleic acid (C).

The nucleic acid probe set according to the present invention as shown in FIG. 1 is designed such that, when the nucleic acid probe complex in the present invention and the target nucleic acid (C) have hybridized with each other as shown in FIG. 2, the fluorescent substance (d) in the fluorescent probe (A) and guanine in the nucleic acid, which includes the target nucleic acid (C), interact with each other to change the fluorescent character of the fluorescent substance (d).

The guanine may exist either inside or outside the base sequence region of the target nucleic acid, insofar as it exists in the nucleic acid that includes the target nucleic acid. When the guanine exists in the base sequence of the target nucleic acid and forms a base pair with cytosine in the hybridized binding probe (B), the interaction between the fluorescent substance (d) and the guanine is somewhat weaker although no particular problem arises. When the guanine forms no base pair for such a reason that the guanine exists outside the base sequence region of the target nucleic acid, on the other hand, the interaction between the fluorescent substance (d) and the guanine is facilitated. Accordingly, the latter situation is more preferred.

Referring next to FIG. 3, a description will be made about conditions under which the fluorescent substance (d) and a desired nucleotide (hereinafter abbreviated as "the nucleotide ε"), which exists in the nucleic acid including the target nucleic acid (C) and has a guanine base, can interact with each other when the nucleic acid probe complex in the present invention and the target nucleic acid (C) have hybridized with each other. It is to be noted that a nucleotide, which has a guanine base at the 3' -end of the target nucleic acid (C), is the nucleotide ε in the case of the description to be made hereinafter.

FIG. 3 shows, on an enlarged scale, an area around the fluorescent substance (d) in FIG. 2. The conditions under which the fluorescent substance (d) and the nucleotide ε can interact with each other depend *inter alia* on the length of a below-described spacer that connects the fluorescent substance (d) and the nucleotide (a) labeled with the fluorescent substance (d) each other, and cannot be specified. Generalizing these conditions, however, they can be defined as will be described hereinafter.

The fluorescent probe (A) is now assumed to have hybridized with the binding probe (B). Base pairs are formed between the fluorescent probe binding region (b1) and the fluorescent probe (A). A nucleotide, which exists in the fluorescent probe binding region (b1) and is closest to the target nucleic acid binding region (b2), will hereinafter be called "the nucleotide α". A nucleotide, which exists in the fluorescent probe (A) and forms a base pair with the nucleotide α will be designated as "β". The distance (β-a) between the nucleotide β and the nucleotide (a) labeled with the fluorescent substance will be designated as "X". In FIG. 3, the nucleotide (α) is a nucleotide having a thymine base located at the 3' -end in the fluorescent probe binding region (b1), while the nucleotide β is a nucleotide which has an adenine base located adjacent the nucleotide (a), which exists in the fluorescent probe (A) and which is labeled with the fluorescent substance (d). X in this case is 1. It is to be noted that an adjacent nucleotide is counted as "X=1" and a nucleotide located adjacent with a base interposed therebetween is counted as "X=2".

On the other hand, base pairs are formed between the target nucleic acid (C) and the target nucleic acid binding region (b2). A nucleotide which exists in the target nucleic acid binding region (b2) and is closest to the nucleotide α will be designated as "the nucleotide γ". The distance (γ-α) between the nucleotide γ and the nucleotide α will be designated as "Y" expressed in terms of the number of base(s). In FIG. 3, the nucleotide γ is a nucleotide having a cytosine base located at the 5' -end in the target nucleic acid binding region (b2), and Y in this case is 1. It is to be noted that the counting method of Y is the same as X.

The binding probe (B) and the target nucleic acid (C) are now assumed to have hybridized with each other. A nucleotide in the target nucleic acid (C), said nucleotide forming a base pair with the nucleotide γ, will be designated as "δ". The distance (δ-ε) between this nucleotide δ and the above-described nucleotide ε will be designated as "Z" expressed in terms of the number of base(s). In FIG. 3, the nucleotide δ is a nucleotide having a guanine base located at the 3' -end in the target nucleic acid (C). As this nucleotide is the nucleotide ε, Z is 0. It is to be noted that the counting method of Z is also the same as X.

As conditions for permitting interaction between the fluorescent substance (d) and the guanine in the nucleotide ε when the nucleic acid probe complex in the present invention and the target nucleic acid (C) have hybridized with each other, the sum of X, Y and Z may preferably be 6 or smaller. The sum of X, Y and Z may be more preferably 4 or smaller, with 2 being most preferred, although it also depends on the length of the spacer connecting the fluorescent substance (d) and the nucleotide (a) labeled with the fluorescent substance (d).

Usable as the fluorescent substance (d) for labeling the fluorescent probe (A) in the present invention is a fluorescent substance (d) which changes in fluorescent character upon interaction with guanine. In the present invention, the term "fluorescent character"means fluorescence intensity, the expression "guanine and the fluorescent substance interact with each other to change the fluorescent character of the fluorescent substance" means that the fluorescence intensity of the fluorescent substance in a state that guanine and the fluorescent substance are not interacting each other is different from its fluorescence intensity in a state that they are interacting each other, and on the extent of this difference, no limitation shall be imposed. Further, the term "quenched or quenching" of fluorescence means that upon interaction of a fluorescent substance with guanine, the fluorescence intensity decreases compared with the fluorescence intensity when the fluorescent substance is not interacting with guanine, and on the extent of this decrease, no limitation shall be imposed.

Examples of fluorescent substances, which can be suitably used in the nucleic acid probe set according to the present invention, include fluorescein and its derivatives [e.g., fluorescein-4-isothiocyanate (FITC),tetrachlorofluorescein,hexachlorofluorescein, tetrabromosulfonefluorescein (TBSF), and derivatives thereof], EDANS (5-(2-aminoethyl)amino-1-naphthalenesulfonic acid), 6-JOE, Alexa Fluor 488 (Molecular Probe Corporation), Alexa Fluor 532 (Molecular Probe Corporation), Cy3 (Amersham BiosciencesCorp.),Cy5(Amersham Biosciences Corp.), Pacific Blue (Molecular Probe Corporation), rhodamine 6G (R6G) and its derivatives [e.g., carboxyrhodamine 6G (CR6G), tetramethylrhodamine (TMR), tetramethylrhodamine isothiocyanate (TMRITC), x-rhodamine, carboxytetramethylrhodamine (TAMRA)], Texas Red (Molecular Probe Corporation), BODIPY-FL (Molecular Probe Corporation), BODIPY-FL/C3 (Molecular Probe Corporation), BODIPY-FL/C6 (Molecular Probe Corporation), BODIPY-5-FAM (Molecular Probe Corporation), BODIPY-TMR (Molecular Probe Corporation), BODIPY-TR (Molecular Probe Corporation), BODIPY-R6G (Molecular Probe Corporation), BODIPY564 (Molecular Probe Corporation), and BODIPY581 (Molecular Probe Corporation).

Of these, the use of fluorescein, fluorescein-4-isothiocyanate, tetrachlorofluorescein, hexachlorofluorescein, tetrabromosulfonefluorescein, EDANS, 6-JOE, Alexa Fluor 488, Alexa Fluor 532, Pacific Blue, rhodamine 6G, carboxyrhodamine 6G, tetramethylrhodamine, carboxytetramethylrhodamine or BODIPY-FL is more preferred, and the use of BODIPY-FL is most preferred.

Concerning the fluorescent probe (A) for use in the nucleic acid probe set according to the present invention, its production can rely upon a custom oligonucleotide synthesis service company (for example, Tsukuba Oligo Service Co., Ltd., Ibaraki, Japan) or the like. No particular limitation is imposed on the method for labeling the fluorescent substance on the oligonucleotide, and a conventionally-known labeling method can be used (Nature Biotechnology, 14, 303-308, 1996; Applied and Environmental Microbiology, 63, 1143-1147, 1997; Nucleic Acids Research, 24, 4532-4535, 1996).

When desired to couple a fluorescent substance, for example, to the 5'-terminal nucleotide, it is necessary to first introduce, for example, $-(CH_2)_n-SH$ as a spacer to a 5' -terminal phosphate group in a manner known *per se* in the art. As such a spacer, a commercial spacer can be used (for example, Midland Certified Reagent Company, U.S.A). In this case, n may stand for 3 to 8, with 6 being preferred. By coupling a fluorescent substance having SH reactivity or its derivative to the spacer, a fluorescently-labeled oligonucleotide can be obtained. The fluorescently-labeled oligonucleotide can be purified by reverse phase chromatography or the like to provide the fluorescent probe (A) for use in the present invention.

Further, a fluorescent substance can also be coupled to the 3'-terminal nucleotide of the oligonucleotide. In this case, it is necessary to introduce, for example, $-(CH_2)_n-NH_2$ as a spacer to the OH group on the C at the 3' site of ribose or deoxyribose. As such a spacer, a commercial spacer can also be used (for example, Midland Certified Reagent Company, U.S. A) As an alternative method, it is also possible to introduce a phosphate group to the OH group on the C at the 3' site of ribose or deoxyribose and then to introduce, for example, $-(CH_2)_n-SH$ as a spacer to the OH group in the phosphate group. In this case, n may stand for 3 to 8, with 4 to 7 being preferred.

By coupling a fluorescent substance, which has reactivity to an amino group or SH group, to the above-described spacer, an oligonucleotide labeled with the fluorescent substance can be synthesized. The oligonucleotide can be purified by reverse phase chromatography or the like to provide the fluorescent probe (A) for use in the present invention. When desired to introduce $-(CH_2)_n-NH_2$ as a spacer, it is convenient to use a kit reagent (for example, Uni-link aminomodifier, Clonetech Laboratories, Inc.). The fluorescent substance can then be coupled to the oligonucleotide in a manner known *per se* in the art.

The nucleotide (a) in the fluorescent probe (A), said nucleotide (a) being labeled with the fluorescent substance, is not limited to one of both terminal nucleotides in the oligonucleotide, and a nucleotide other than both the terminal nucleotides can be labeled with the fluorescent substance (ANALYTICAL BIOCHEMISTRY, 225, 32-38, 1998).

The fluorescent probe (A) that constitutes the nucleic acid probe set according to the present invention can be prepared as described above. The preferred form of the fluorescent probe (A) is, however, one with the 5'-terminal or 3'-terminal nucleotide being labeled with the fluorescent substance (d), and more preferred is one with the 5'-terminal nucleotide being labeled with BODIPY-FL. As the base in the nucleotide labeled with the fluorescent substance (d), guanine or cytosine is preferred.

The fluorescent probe (A) that constitutes the nucleic acid probe set according to the present invention is only needed to have a base sequence which can hybridize with the fluorescent probe binding region (b1) in the binding probe (B), and no particular limitation is imposed on its base length. However, a length of 4 bases or less may not be preferred in that it may lead to a lower hybridization efficiency, and a length of 51 bases or more may not be preferred either in that it tends to form non-specific hybrids. Therefore, the fluorescent probe (A) may be preferably 5 to 50 bases long, more preferably 10 to 35 bases long, especially preferably 12 to 30 bases long.

The base sequence of the fluorescent probe (A) may include one or more nucleotides which are not complementary to the corresponding one or ones in the fluorescent probe binging region (b1), insofar as the fluorescent probe (A) can hybridize with the fluorescent probe binging region (b1) in the binding probe (B). Like the fluorescent probe (A) shown in FIG. 1, for example, one having at the 5' -end thereof a nucleotide which is not complementary to the corresponding one in the binding probe (B) may be also usable. On the other hand, the base sequence of the fluorescent probe binding region (b1) in the binding probe (B) is not particularly limited insofar as it can hybridize with the fluorescent probe (A), and its base length depends on the base length of the fluorescent probe (A).

The target nucleic acid binding region (b2) in the binding probe (B) is only needed to have a base sequence which can hybridize with the target nucleic acid (C). The base length of the target nucleic acid binding region (b2) depends on the base length of the target nucleic acid. However, a length of 4 bases or less may not be preferred in that it may lead to a lower efficiency of hybridization with the target nucleic acid (C), and a length of 61 bases or more may not be preferred either in that it tends to form non-specific hybrids. Therefore, the target nucleic acid binding region (b2) may be preferably 5 to 60 bases long, more preferably 15 to 30 bases long. The target nucleic acid binding region (b2) may include a base sequence that forms no base pair with the target nucleic acid (C), insofar as it can hybridize with the target nucleic acid (C).

The nucleic acid probe set according to the present invention can be used in various analysis methods of nucleic acids. A description will hereinafter be made of an illustrative detection method of a target nucleic acid, which uses the nucleic acid probe set according to the present invention to determine whether or not the target nucleic acid exists in a solution.

A solution, which is to be detected for the target nucleic acid and will hereinafter be called "the detection sample", is first serially diluted to prepare several kinds of solutions. The nucleic acid probe set according to the present invention, in other words, the fluorescent probe (A) and binding probe (B) are added in a constant amount to these serially-diluted detection samples. After the solutions are adjusted in temperature such that the thus-added nucleic acid probe complex in the present invention and the target nucleic acid can hybridize with each other, the solutions are measured for fluorescence intensity. The temperature, at which the probe complex in the present invention and the target nucleic acid are subjected to hybridization with each other, varies depending on the melting temperature (hereinafter called "Tm1") of the hybridized complex of the nucleic acid probe complex in the present invention and the target nucleic acid and other solution conditions. However, the hybridization temperature may be preferably in a temperature range where sequence-specific hybridization takes place between the nucleic acid probe complex and the target nucleic acid but non-specific hybridization does not occur between them, more preferably Tm1 to (Tm1 - 40)°C, still more preferably Tm1 to (Tm1 - 20)°C, even still more preferably Tm1 to (Tm1 - 10)°C. As one example of such a preferred temperature, about 60°C can be mentioned.

The melting temperature (hereinafter called "Tm2") of the complex of the fluorescent probe and binding probe, which constitute the nucleic acid probe set according to the present invention, may be preferably higher than Tm1, with (Tm2 - Tm1) of 5°C or greater being more preferred, to assure the measurement of the fluorescence intensity.

When the target nucleic acid does not exist in the detection sample, a similar fluorescence intensity is observed from each of the serially-diluted detection samples. When the target nucleic acid exists in the detection sample, on the other hand, fluorescence from the fluorescent substance in the nucleic acid probe set according to the present invention is quenched by guanine in the nucleic acid which includes the target nucleic acid. The degree of this quenching varies by changing the ratio of the nucleic acid probe set to the target nucleic acid in the solution. By adding the nucleic acid probe set according to the present invention to detection samples, which has been serially diluted as mentioned above, and measuring their fluorescence intensities, it is, therefore, possible to determine the existence/non-existence of the target nucleic acid from the occurrence/non-occurrence of a fluorescence quenching and also to quantify the existing amounts of the target nucleic acid from the magnitudes of the fluorescence quenching.

The nucleic acid probe set according to the present invention can also be used in a real-time PCR method. When quantification of an amplification product is desired by using the nucleic acid probe set according to the present invention in the real-time PCR method, a base sequence to be amplified by PCR or a portion thereof is chosen as a target nucleic acid, and the base sequence of the target nucleic acid binding region (b2) in the binding probe (B) is determined such that the target nucleic acid binding region (b2) can hybridize with the target nucleic acid.

The nucleic acid probe set according to the present invention, which has been prepared as described above, is added to a PCR reaction solution, a PCR reaction is conducted, and the fluorescence intensity is measured in each cycle of PCR. When the target nucleic acid in the reaction solution is amplified through the PCR reaction, the fluorescence from the fluorescent substance in the nucleic acid probe set according to the present invention is quenched by guanine in a nucleic acid that includes the target nucleic acid. The amplification product by PCR can, therefore, be quantified from the fluorescence intensity and the degree of the fluorescence quenching.

The nucleic acid probe set according to the present invention can also be used in an analysis of a nucleic acid for a base sequence polymorphism. Examples of analyzable polymorphisms include a single nucleotide polymorphism, base substitution, base deletion, base insertion and the like with respect to a base sequence as a reference. One example of such an analysis method will be described hereinafter.

In this analysis method, the reference base sequence is used as a target nucleic acid (C), and a solution containing the target nucleic acid (C) and another solution containing a nucleic acid to be analyzed are prepared. After the nucleic acid probe set according to the present invention, that is, the binding probe (B), which has the target nucleic acid binding region (b2) designed to hybridize with the target nucleic acid (C), and the fluorescent probe (A) are added to the respective solutions, the added nucleic acid probe complex in the present invention is subjected to hybridization with the target nucleic acid (C) and the nucleic acid to be analyzed in the respective solutions, and the temperature dependences of fluorescence intensities are then measured. Described specifically, while changing the temperature of each solution from a low temperature to a high temperature, the fluorescence intensity is measured at each temperature.

A plot of the measurement results against temperature is called a "melting curve". By differentiating the melting curve of the solution, which contains the target nucleic acid, with respect to temperature, Tm1 of the hybridized complex of the nucleic acid probe complex according to the present invention and the target nucleic acid (C) can be easily determined as a temperature that indicates an extreme value. Such a melting curve analysis can be performed by using a commercial program known well to those skilled in the art.

The fluorescence intensity of the solution, which contains the target nucleic acid (C), is reduced at a low temperature by the fluorescence quenching effect of guanine in the nucleic acid that includes the target nucleic acid (C). When the solution temperature is raised to around Tm1, however, the target nucleic acid dissociates from the nucleic acid probe complex in the present invention, the degree of fluorescence quenching decreases, and therefore, the fluorescence intensity suddenly increases. When there is, in the base sequence of the nucleic acid to be analyzed, a base sequence polymorphism, for example, a single nucleotide polymorphism, base substitution, base deletion, base insertion or the like with respect to the base sequence of the target nucleic acid, Tm1 of the hybridized complex of the nucleic acid to be analyzed and the nucleic acid probe complex in the present invention indicates a value lower than Tm1 of the hybridized complex of the target nucleic acid (C) and the nucleic acid probe complex in the present invention. By comparing the temperature dependence of the fluorescence intensity of the hybridized complex of the target nucleic acid and the nucleic acid probe complex in the present invention with the temperature dependence of the fluorescence intensity of the hybridized complex of the nucleic acid as the analysis target and the nucleic acid probe complex in the present invention, the nucleic acid as the analysis target can, therefore, be analyzed for a base sequence polymorphism with respect to the base sequence of the target nucleic acid. As such an analytical procedure, their melting curves may be compared with each other. However, the existence or non-existence of a mutation can be readily determined by differentiating the respective melting curves with respect to temperature, determining Tm1s as temperatures that give extreme values, and then comparing Tm1s.

When a nucleotide having a guanine base that applies the quenching effect to the fluorescent substance in the fluorescent probe has mutated in the base sequence of the nucleic acid as an analysis target, no decrease occurs in fluorescence intensity by the fluorescence quenching effect at any temperature so that the mutation can be specified from the melting curve.

In a melting curve analysis, it has heretofore been needed to prepare fluorescently-labeled, costly nucleic acid probes of different base sequences specifically for individual target nucleic acids, and therefore, a substantial time has been needed for their synthesis. The use of the nucleic acid probe set according to the present invention in a melting curve analysis can obviate the preparation of fluorescently-labeled, costly nucleic acid probes specifically for individual target nucleic acids, and therefore, can reduce the preparation time for the melting curve analysis and can more economically perform the melting curve analysis.

The above-described nucleic acid probe set according to the present invention is composed of the binding probe (B), which has the one fluorescent probe binding region (b1), and the one fluorescent probe (A). As an alternative, the nucleic acid probe set according to the present invention may have two fluorescent probe binding regions (b1) as shown in FIG. 8. These fluorescent probe binding regions (b1) may have the same base sequence or different base sequences, although the different base sequences are preferred. In this case, a fluorescent probe may be used singly, but it is preferred to have two fluorescent probes (A) of different base sequences. The constructions of the fluorescent probe binding regions (b1) and fluorescent probes (A) in the nucleic acid probe set according to the present invention as shown in FIG. 8 are the same as described above.

The above-described nucleic acid probe set according to the present invention, in which the binding probe (B) has the two fluorescent probe binding regions of different base sequences, can be suitably used as a replacement for a conventionally-used AB probe in an ABC-PCR (Alternately Binding Probe Competitive PCR; see Tani et al., Analytical Chemistry, Preprint) method.

The ABC-PCR method disclosed by Tani et al. is a sort of competitive PCR method, and is a method that conducts a PCR reaction by adding a nucleic acid (hereinafter called "the internal standard nucleic acid") and a nucleic acid probe (hereinafter called "the AB probe") to a PCR reaction solution in addition to a nucleic acid that contains a base sequence to be amplified (hereinafter called "the amplification sequence"). The internal standard nucleic acid has a similar base sequence as the amplification sequence except that a portion of the base sequence has been replaced to guanine. The AB probe has a base sequence complementary to a portion of the amplification sequence and is labeled at opposite ends thereof with different fluorescent substances which are different in excitation wavelength and fluorescence wavelength. The AB probe is designed such that, when hybridized with the nucleic acid containing the amplification sequence, only one of the fluorescent substances is quenched by the guanine in the nucleic acid and that, when hybridized with the internal standard nucleic acid, both of the fluorescent substances are quenched by the guanine in the internal standard nucleic acid. An amplification product of the PCR reaction can, therefore, be quantified from the fluorescence intensities of the two kinds of fluorescent substances.

As fluorescently-labeled AB probes for use in the above-described conventional ABC-PCR method, different types of probes have to be used specifically for individual base sequences to be amplified. The use of nucleic acid probe sets according to the present invention in place of the above-described AB probes can obviate the need to prepare different types of fluorescently-labeled, costly fluorescent probes specifically for individual base sequences to be amplified, and therefore, can perform the ABC-PCRmethod more economically.

One example of nucleic acid probe sets according to the present invention, which are suited for use in such an ABC-PCR method, is illustrated in FIG. 8. In this case, a portion of a base sequence to be amplified by PCR is chosen as a target nucleic acid (C), and the base sequence of the target nucleic acid binding region (b2) in the binding probe (B) is determined such that the target nucleic acid binding region (b2) can hybridize with the target nucleic acid. When the nucleic acid probe set according to the present invention is used as a replacement for an AB probe in the ABC-PCR method, the combination of BODIPY-FL and TAMRA can be mentioned as a preferred example of the combination of the fluorescent substances which are to be labeled to the two fluorescent probes constituting the nucleic acid probe set and are different in excitation wavelength and fluorescence wavelength.

## Examples

The present invention will next be described more specifically based on examples. However, the following examples are merely illustrative of the present invention, and are not intended to be limiting the present invention.

Example 1 Target Nucleic Acid Detection Test

Using a nucleic acid probe set for an oligonucleotide, which had the same base sequence as a portion of the human UCP1 gene, as a target nucleic acid (SEQ ID NO: 1), a target nucleic acid detection test was performed. As is shown in FIG. 1, the nucleic acid probe set was composed of a binding probe (SEQ ID NO:2) and a fluorescent probe. The binding probe had, on the side of a 5' -end thereof, (b1) a fluorescent probe binding region of a base sequence complementary to the fluorescent probe, and on the side of a 3' -end thereof, (b2) a target nucleic acid binding region of a base sequence complementary to the target nucleic acid. The fluorescent probe had a base sequence (SEQ ID NO:3), and was labeled at a 5' -terminal nucleotide thereof with BODIPY-FL (Molecular Probe Corporation).

SEQ ID NO:1 tgaccacagtttgatcgagtg
SEQ ID NO:2 tctcggagtccgggcgatcactcgatcaaactgtggtca -phosphate
SEQ ID NO:3 BODIPY-FL-catcgcccggactccgaga-phosphate

The binding probe and fluorescent probe both had a phosphate group at 3' ends thereof. Syntheses of the target nucleic acid, binding probe and fluorescent probe were relied upon Tsukuba Oligo Services Co., Ltd. (Tsukuba, Japan).

Six kinds of target nucleic acid solutions were prepared by serially diluting the target nucleic acid in a PCR buffer (which contained 50 mM KCl, 10 mM Tris-HCl and 1.5 mM $MgCl_2$; the concentrations were all final concentrations; pH 8.7) such that the final concentration of the target nucleic acid became 0, 0.1, 0.2, 0.3, 0.4 and 0.5 μM. To the respective target nucleic acid solutions, the fluorescent probe (final concentration: 0.05 μM) and binding probe (final

concentration: 0.2 μM) were added. Each solution was measured at 60°C for fluorescence intensity. For the measurement of fluorescence intensity, LightCycler® 480 (Roche Diagnostics K.K.) was used. The measurement results are shown in FIG. 4.

It has been confirmed from FIG. 4 that, compared with a case that a target nucleic acid does not exist in a solution, the fluorescence intensity decreases in a case that the target nucleic acid exists in the solution and this decrease in fluorescence intensity is greater as the concentration of the target nucleic acid in the solution is higher. These results indicate that the existence of a target nucleic acid having a specific base sequence can be determined by adding the nucleic acid probe set according to the present invention to a solution of the target nucleic acid and measuring the fluorescence intensity of the solution, and also suggest that this measurement can be applied to the quantification of the target nucleic acid in the solution.

Example 2 Quantitative Real-time PCR Experiment Prepared were five kinds of reaction solutions for real-time PCR, which contained 500 ng, 50 ng, 5 ng, 0.5 ng and 0.05 ng of a human genomic DNA sample (Promega K.K.), respectively, as a template DNA for PCR. Those reaction solutions each contained TITANIUM Taq DNA polymerase (Clonetech Laboratories, Inc.) as a DNA polymerase, four kinds of dNTPs (each at 0.2 mM final concentration), a forward primer (SEQ ID NO : 4, final concentration: 0.5 μM), a reverse primer (SEQ ID NO:5, final concentration: 0.15 μM), a predetermined amount of TITANUM Taq PCR buffer (Clonetech Laboratories, Inc.), and a nucleic acid probe set according to the present invention.

The nucleic acid probe set according to the present invention was for a portion (SEQ ID NO:1) of the UCP1 gene in the human genome as a target nucleic acid, and was composed of a binding probe (SEQ ID NO:2; final concentration: 0.2 μM) and a fluorescent probe (final concentration: 0.05 μM). The binding probe had, on the side of a 5' -end thereof, (b1) a fluorescent probe binding region of a base sequence complementary to the fluorescent probe, and on the side of a 3' -end thereof, (b2) a target nucleic acid binding region of a base sequence complementary to the target nucleic acid. The fluorescent probe had the base sequence (SEQ ID NO:3), and was labeled at a 5' -terminal nucleotide thereof with BODIPY-FL (Molecular Probe Corporation). Each PCR reaction solution was prepared by adding sterilized water to bring its volume to 20 μL. A nucleic acid including the target nucleic acid was amplified by a PCR reaction as will be described hereinafter.

SEQ ID NO:4 agtggtggctaatgagagaa
SEQ ID NO:5 aaggagtggcagcaagt

The binding probe and fluorescent probe both had a phosphate group at 3' ends thereof. Synthesis of the nucleic acid probe was relied upon Tsukuba Oligo Service Co. , Ltd. (Tsukuba, Japan), and syntheses of the forward primer and reverse primer were relied upon Nihon Gene Research Laboratories, Inc. (Sendai, Japan).

Using a real-time PCR system (LightCycler® 480, Roche Diagnostics K.K.), the reaction solutions were subjected to the following PCR reaction.

(1) Thermal denaturation step: 95°C, 120 seconds
(2) Thermal denaturation step: 95°C, 10 seconds
(3) Annealing step: 60°C, 20 seconds
(4) Extension step: 72°C, 10 seconds

After the thermal reaction step (1), the steps (2) to (4) were repeated. In each annealing step (3), the fluorescence intensity was measured to determine the cycle number (hereinafter abbreviated as "Ct (cycle threshold) value") in which the fluorescence intensity first decreased to a value of 95% or smaller of the fluorescence strength in the $5^{th}$ cycle. It is to be noted that the Ct value is a value determined from a line drawn by connecting the fluorescence intensities in the respective PCR cycles.

A diagram of plots of Ct values (an average of two measurements, each) versus the respective amounts of the template DNA added initially is shown in FIG. 5. It has been found from FIG. 5 that a good correlation exists between amounts of a template DNA added initially and Ct values measured by using the nucleic acid probe set according to the present invention. Therefore, the results of this experiment indicate that in a real-time PCR method, the nucleic acid probe set according to the present invention is very useful as a probe for quantifying a template DNA including a target nucleic acid and contained in a reaction solution before a PCR reaction and an amplification product including the target nucleic acid and contained in the reaction solution after the PCR reaction.

Example 3 Melting Curve Analysis of Complex of Nucleic Acid Probe Set According to Present invention and Target Nucleic Acid

Using a nucleic acid probe set according to the present invention for the nucleic acid of 21 bases long (SEQ ID NO:1), which was the portion of the human UCP1 gene, as a target nucleic acid, a melting curve analysis of a complex of the nucleic acid probe set according to the present invention and the target nucleic acid was performed. The nucleic acid probe set was composed of a binding probe (SEQ ID NO:2) and a fluorescent probe. The binding probe had, on the side of a 5' -end thereof, a region of a base sequence complementary to the fluorescent probe, and on the side of a 3' -end thereof, a region of a base sequence complementary to the target nucleic acid (SEQ ID NO: 1). The fluorescent probe had the base sequence (SEQ IDNO: 3) and was labeled at a 5' -terminal nucleotide thereof with BODIPY-FL (Molecular Probe Corporation). Using, in place of the above-described target nucleic acid, a similar nucleic acid (SEQ IQ NO: 6) as the above-described target nucleic acid except that the $17^{th}$ base from the 5' -end had been substituted to adenine, another melting curve analysis was performed likewise. It is to be noted that in the subsequent part of Example 3, the target nucleic acid and the nucleic acid (SEQ ID NO:6) will be collectively called "the target nucleic acids".
SEQ ID NO:6 tgaccacagtttgatcaagtg

The binding probe and fluorescent probe both had a phosphate group at 3' ends thereof. Syntheses of the target nucleic acids and nucleic acid probe were relied upon Tsukuba Oligo Services Co., Ltd. (Tsukuba, Japan).

Three kinds of solutions were provided, including solutions prepared by adding the target nucleic acid and the nucleic acid (SEQ ID NO: 6) (each, to 0.1 $\mu$M final concentration) to portions of the PCR buffer which contained the nucleic acid probe set according to the present invention (the fluorescent probe (final concentration: 0.05 $\mu$M) and the binding probe (final concentration: 0.2 $\mu$M)), respectively, and a further solution which was a portion of the PCR buffer without addition of any of the target nucleic acids. The PCR buffer contained KCl, Tris-HCl and $MgCl_2$ at final concentrations of 50 mM, 10 mM and 1.5 mM, respectively, and had pH 8.7. After each solution was maintained at 40°C for 60 seconds to hybridize the nucleic acid probe set according to the present invention and, if any, the corresponding one of the target nucleic acids, the fluorescence intensity was measured while raising the solution temperature from 40°C to 80°C (the measurement was conducted 5 times at every Celsius degree). As a result, the melting curves shown in FIG. 6 were obtained. In the figure, the melting curve of the solution containing the target nucleic acid (SEQ ID NO:1) is indicated by D, the solution containing the nucleic acid (SEQ ID NO:6) is indicated by E, and the solution free of the target nucleic acids is indicated by F. Further, negative first differential curves of these melting curves are shown in FIG. 7. It is to be noted that LightCycler® 480 (Roche Diagnostics K. K.) was used for the measurement of the fluorescence intensity.

As a result of the experiment, the performance of the melting curve analysis by the nucleic acid probe set according to the present invention made it possible to clearly determine, from the negative first differential curves of the melting curves indicated by D and E in FIG. 7, the temperatures at which Tm1s of the complexes of the nucleic acid probe set according to the present invention and the target nucleic acids had extreme minima, respectively. Tm1 of the complex of the target nucleic acid (SEQ ID NO:1), which was completely complementary to the target nucleic acid binding region, and the nucleic acid probe set was about 64°C, while Tm1 of the complex of the nucleic acid (SEQ ID NO: 6), which had a base pair mismatched to the target nucleic acid binding region, and the nucleic acid probe set was lower, specifically about 56°C. These results indicate that the nucleic acid probe set according to the present invention can be suitably used as a replacement for conventionally-used nucleic acid probes in melting curve analyses.

Example 4 ABC-PCR Experiment

Using a nucleic acid probe set according to the present invention for a portion (SEQ ID NO: 7) of the amoA gene coding an ammonia monooxygenase of an ammonium oxidizing bacteria (*Nitrosomonas europaea* (NBRC 14298)) as a target nucleic acid, an ABC-PCR experiment was performed, and the effectiveness of the nucleic acid probe set according to the present invention was evaluated.
SEQ ID NO:7 gtcaccagccagttgcgtgtcagat

The nucleic acid probe set for use in this ABC-PCR experiment was composed of one binding probe and two fluorescent probes as shown in FIG. 8. The binding probe (SEQ ID NO:8) was an oligonucleotide of 55 bases long, which had two fluorescent probe binding regions of different base sequences on the side of a 5'-end and on the side of a 3' -end, respectively, and had a target nucleic acid binding region in a central part. The fluorescent probe (Al-amoA) was designed to hybridize with the side of the 5' -end of the binding probe, was an oligonucleotide of 15 bases long, which had a base sequence (SEQ ID NO:9), was labeled at a 5'-terminal nucleotide thereof with BODIPY-FL (Molecular Probe Corporation), and had a phosphate group at the 3' -end.

On the other hand, the fluorescent probe (A2-amoA) was designed to hybridize with the side of the 3' -end of the binding probe, and was an oligonucleotide of 15 bases long. The oligonucleotide had a base sequence (SEQ ID NO : 10) and was labeled at a 3' -terminal nucleotide thereof with carboxytetramethylrhodamine (TAMRA).

SEQ ID NO:8
attcgggcgggcttaatctgacacgcaactggctggtgactttgggggggg gttt
SEQ ID NO:9 BODIPY-FL-taagcccgcccgaat-phosphate

SEQ ID NO:10 aaacccccccccaaa-TAMRA

Used as an internal standard nucleic acid in this example was a similar amoA gene as the amoA gene of the *Nitrosomonas europaea* (NBRC 14298), the latter amoA gene having the above-described target nucleic acid, except that the two bases on the side of the 3' -end (on an outer side), said two bases being located adjacent the 3' -end of the target nucleic acid, were replaced to guanine in a manner known *per se* in the art (see, for example, Higuchi et al. , NucleicAcids Res. , 16, 7351-5367, 1988). The fluorescent probe (Al-amoA) was not quenched when hybridized as a nucleic acid probe complex with the target nucleic acid, but was quenched by the substituted guanine in the similar amoA gene when hybridized with the internal standard nucleic acid.

A PCR reaction solution used in the ABC-PCR experiment contained a predetermined amount of TITANIUM Taq DNA polymerase (Clonetech Laboratories, Inc.) as a DNA polymerase, a predetermined amount of TITANIUM Taq PCR buffer (Clonetech Laboratories, Inc.), dATP, dCTP and dGTP (each, final concentration: 200 μM), dUTP (Roche Diagnostics K.K.) (final concentration: 600 μM), a forward primer (SEQ ID NO: 11, final concentration: 0.1 μM), a reverse primer (SEQ ID NO: 12, final concentration: 1 μM), the binding probe (final concentration: 0.1 μM) and the two types of fluorescent probes (each, final concentration: 0.15 μM), uracil-DNA glycosylase (0.25 U, Roche Diagnostics K.K.) for avoiding contamination by an amplification product, and $10^3$ copies of the internal standard nucleic acid. A PCR reaction solution free of the above-descried target nucleic acid and PCR reaction solutions containing 10, 100, 300, 1,000, 3,000, 10,000 and 100,000 copies of the above described target nucleic acid, respectively, were prepared, and sterilized water was added to the respective PCR reaction solutions to bring their volumes to 25 μL.

SEQ ID NO:11 gghgactgggayttctgg
SEQ ID NO:12 cctckgsaaagccttcttc

In the above-described sequences, h means A, C or T, y means C or T, k means G or T, and s means G or C.

Using Bio101 FastDNA spin kit (Qbiogene, Inc.), a DNA was extracted from 0.5 g (wet weight) of an ammonium oxidizing bacteria (*Nitrosomonas europaea* (NBRC14928)) (purchased from Biological Research Center, National Institute of Technology and Evaluation (NITE)) cultured in NBRC liquid medium. Using Microcon YM-50 (Millipore Corporation), the DNA was purified. The purified DNA was used as the above-described target nucleic acid.

To avoid contamination, during the preparation of the PCR reaction solutions, the solutions were maintained at room temperature and were subjected to a glycosylase reaction by uracil-DNA glycosylase. After the preparation of the solutions, they were subjected to the following PCR reaction.

(1) Thermal denaturation step: 95°C, 120 seconds
(2) Thermal denaturation step: 95°C, 45 seconds
(3) Annealing step: 55°C, 60 seconds
(4) Extension step: 72°C, 45 seconds
(5) Extension step: 72°C, 45 seconds

After the thermal reaction step (1), the steps (2) to (4) were repeated 60 cycles, and subsequently, the extension step (5) was conducted. As a thermal cycler for the PCR reaction, iCycler (Bio-Rad Laboratories, Inc.) was used.

After completion of the PCR reaction, the fluorescence intensity of each solution was measured at 95°C and 55°C by using SmartCycler® (Cepheid). The fluorescence intensity of the fluorescence probe Al-amoA (hereinafter called "the fluorescence intensity Al") was measured in an excitation wavelength range of 450 to 495 nm and in a detection wavelength range of 505 to 537 nm, while the fluorescence intensity of the fluorescence probe A2-amoA (hereinafter called "the fluorescence intensity A2") was measured in an excitation wavelength range of 527 to 555 nm and in a detection wavelength range of 565 to 605 nm.

The resulting fluorescence intensities were introduced into the following formula (1) to determine the corrected fluorescence intensity values with respect to the seven kinds of reaction solutions that contained the target nucleic acid.

$$\text{Corrected fluorescence intensity value} = [(G_{U,55}/G_{U,95}) - (G_{55}/G_{95})]/[(R_{U,55}/R_{U,95}) - (R_{55}/R_{95})] \quad (1)$$

where,

$G_{U,55}$: Fluorescence intensity Al at 55°C of the reaction solution free of the target nucleic acid.
$G_{u,95}$: Fluorescence intensity Al at 95°C of the reaction solution free of the target nucleic acid.

$G_{55}$: Fluorescence intensity Al at 55°C of the reaction solution containing the target nucleic acid.
$G_{95}$: Fluorescence intensity Al at 95°C of the reaction solution containing the target nucleic acid.
$R_{U,55}$: Fluorescence intensity A2 at 55°C of the reaction solution containing the target nucleic acid.
$R_{u,95}$: Fluorescence intensity A2 at 95°C of the reaction solution containing the target nucleic acid.
$R_{55}$: Fluorescence intensity A2 at 55°C of the reaction solution free of the target nucleic acid.
$R_{95}$: Fluorescence intensity A2 at 95°C of the reaction solution free of target nucleic acid.

A diagram of the resulting corrected fluorescence intensity values as plotted on a semi-logarithmic graph, in which the copy number of the target nucleic acid before the PCR reaction are plotted along the abscissas, is shown in FIG. 9. It has been found from this diagram that, even when an ABC-PCR experiment is performed by using the nucleic acid probe set according to the present invention, a graph (relational expression) that becomes a rectangular hyperbola can be obtained similarly as in the case that a conventionally-employed AB probe is used.

Using this relational expression as a calibration curve, an experiment was next conducted to calculate the copy numbers of the target nucleic acid, which were contained in reaction solutions before a PCR reaction, from the fluorescence intensities of the reaction solutions after the ABC-PCR reaction.

Prepared were three kinds of PCR reaction solutions, which were similar to the above-described ABC-PCR reaction solution except for the number of copies of the target nucleic acid. As the reaction solutions, solutions containing 0, 2,000 and 6,000 copies of the target nucleic acid, respectively, were prepared as many as 5 samples per solution. The three kinds of PCR reaction solutions were subjected to a 60-cycle PCR reaction as in the above-described ABC-PCR experiment, the fluorescence intensities after the reaction were introduced into the formula (1) to determine the corrected fluorescence intensity values, and by applying these values to the above-described calibration curve, the copy numbers of the target nucleic acid before the PCR reaction were calculated. The results are shown in Table 1.

Table 1

| Number of copies of target nucleic acid before PCR reaction | 2,000 | 6,000 |
|---|---|---|
| 1st calculated value | 2,400 | 5, 900 |
| 2nd calculated value | 1, 900 | 5,800 |
| 3rd calculated value | 2,200 | 6,500 |
| 4th calculated value | 2,300 | 6,200 |
| 5th calculated value | 1,800 | 5, 900 |
| Average of calculated values | 2,120 | 6,060 |
| Standard deviation (%) of calculated values | 12.210 | 4.754 |

As is clearly envisaged from Table 1, the calculated value of the number of copies of the target nucleic acid before the PCR reaction as calculated from the fluorescence intensity of each reaction solution after the PCR reaction conformed closely to the number of copies of the target nucleic acid actually used. This result indicates that similar to the conventionally-used AB probes, the nucleic acid probe set according to the present invention can also be used in an ABC-PCR experiment and has high reliability.

**Industrial Applicability**

The present invention can provide a nucleic acid probe set which can specifically analyze a target nucleic acid. The nucleic acid probe set according to the present invention does not require preparing a fluorescently-labeled, costly nucleic acid probe specifically for every target nucleic acid to be analyzed, and therefore, has an advantage that a nucleic acid probe for a target nucleic acid can be prepared at low cost and in a short time compared with the conventional nucleic acid probes. The nucleic acid probe set according to the present invention can be applied to the detection, quantification and polymorphism analysis of nucleic acids, the detection of mutations, and the like in fields such as medical science, molecular biology and agricultural science.

**Brief Description of the Drawings**

FIG. 1] FIG. 1 is a schematic diagram showing a nucleic acid probe complex in the present invention before hybridization with a target nucleic acid (C).

[FIG. 2] FIG. 2 is a schematic diagram showing the nucleic acid probe complex in the present invention as hybridized with the target nucleic acid (C).

[FIG. 3] FIG. 3 is a schematic diagram showing on an enlarged scale an area around a fluorescent substance (d) in FIG. 2.

[FIG. 4] FIG. 4 is a diagram of plots of the values of fluorescence intensities measured after addition of a constant amount of a nucleic acid probe set according to the present invention to solutions having different target nucleic acid concentrations.

[FIG. 5] FIG. 5 is a diagram of plots of Ct values of PCR reaction solutions, which were different in the amount of a template DNA added initially, as determined by subjecting the solutions to real-time PCR measurement.

[FIG. 6] FIG. 6 is a diagram illustrating melting curves of hybridized complexes of a nucleic acid probe complex in the present invention and target nucleic acids.

[FIG. 7] FIG. 7 is a diagram illustrating negative first differential curves of the melting curves in FIG. 6.

[FIG. 8] FIG. 8 is a schematic diagram illustrating a nucleic acid probe set according to the present invention as used in Example 4.

[FIG. 9] FIG. 9 is a diagram showing correlations between corrected fluorescence intensity values, which were obtained by an ABC-PCR experiment making use of a nucleic acid probe set according to the present invention, and the copy numbers of a target nucleic acid before the reaction.

**Legend**

| | |
|---|---|
| A | Fluorescent probe |
| B | Binding probe |
| C | Target nucleic acid |
| a | Nucleotide labeled with a fluorescent substance |
| b1 | Fluorescent probe binding region |
| b2 | Target nucleic acid binding region |
| d | Fluorescent substance |
| α | Nucleotide α |
| β | Nucleotide β |
| γ | Nucleotide γ |
| δ | Nucleotide δ |
| ε | Nucleotide ε |

## Claims

**1.** A nucleic acid probe set comprising:

(A) one fluorescent probe which is formed of an oligonucleotide including (a) a nucleotide labeled with (d) a fluorescent substance, wherein the nucleotide (a) is a 5'-terminal nucleotide of said oligonucleotide, and
(B) a binding probe formed of an oligonucleotide having (b1) one fluorescent probe binding region, to which the fluorescent probe (A) can hybridize, and (b2) a target nucleic acid binding region which can hybridize to a target nucleic acid (C),

wherein the nucleic acid probe set is designed such that, when the fluorescent probe (A) hybridizes to the fluorescent probe binding region (b1) and the target nucleic acid (C) hybridizes to the target nucleic acid binding region (b2), fluorescence from the fluorescent substance is quenched by guanine in a nucleic acid that includes the target nucleic acid (C), wherein said guanine and the fluorescent substance (d) labeled on the nucleotide (a) interact with each other to change a fluorescent character of the fluorescent substance (d), wherein said fluorescent probe binding region (b1) of binding probe (B) is located on the 5' -end of the binding probe (B).

**2.** A nucleic acid probe set comprising:

(A) one fluorescent probe which is formed of an oligonucleotide including (a) a nucleotide labeled with (d) a fluorescent substance, wherein the nucleotide (a) is a 3'- terminal nucleotide of said oligonucleotide, and
(B) a binding probe formed of an oligonucleotide having (b1) one fluorescent probe binding region, to which the fluorescent probe (A) can hybridize, and (b2) a target nucleic acid binding region, which can hybridize to a target nucleic acid (C),

wherein the nucleic acid probe set is designed such that, when the fluorescent probe (A) hybridizes to the fluorescent probe binding region (b1) and the target nucleic acid (C) hybridizes to the target nucleic acid binding region (b2), fluorescence from the fluorescent substance is quenched by guanine in a nucleic acid that includes the target nucleic acid (C), wherein said guanine and the fluorescent substance (d) labeled on the nucleotide (a) interact with each other to change a fluorescent character of the fluorescent substance (d), wherein said fluorescent probe binding region (b1) of binding probe (B) is located on the 3' -end of the binding probe (B).

**3.** A nucleic acid probe set comprising:

(A) two fluorescent probes, each of which is formed of an oligonucleotide including (a) a nucleotide labeled with (d) a fluorescent substance, wherein the nucleotide (a) of a first fluorescent probe is a 5' -terminal nucleotide of an oligonucleotide of the first fluorescent probe, and the nucleotide (a) of a second fluorescent probe is a 3'-terminal nucleotide of an oligonucleotides of the second fluorescent probe, and
(B) a binding probe formed of an oligonucleotide having (b1) two fluorescent probe binding regions of different base sequences on the side of a 5' end and on the side of a 3' end of said oligonucleotide and to which the fluorescent probes (A) can hybridize, and (b2) a target nucleic acid binding region which can hybridize to a target nucleic acid (C),

wherein the nucleic acid probe set is designed such that, when the fluorescent probes (A) hybridize to the fluorescent probe binding regions (b1) and the target nucleic acid (C) hybridizes to the target nucleic acid binding region (b2), fluorescence from the fluorescent substance is quenched by guanine in a nucleic acid that includes the target nucleic acid (C), whereby the guanine and the fluorescent substance (d) labeled on the nucleotide (a) interact with each other to change a fluorescent character of the fluorescent substance (d).

**4.** The nucleic acid probe set according to any of claims 1 to 3, wherein the fluorescent substance (d) is any one selected from the group consisting of fluorescein, fluorescein-4-isothiocyanate, tetrachlorofluorescein, hexachlorofluorescein, tetrabromosulfonefluorescein, EDANS, 6-JOE, Alexa Fluor 488, Alexa Fluor 532, Pacific Blue, rhodamine 6G, carboxyrhodamine 6G, tetramethylrhodamine, carboxytetramethylrhodamine and BODIPY-FL.

**5.** The nucleic acid probe set according to any one of claims 1 to 3, wherein the one or two fluorescent probe or probes (A) are each 5 to 50 bases long.

**6.** The nucleic acid probe set according to any one of claims 1 to 3, wherein the target nucleic acid binding region (b2) is 5 to 60 bases long.

**7.** The nucleic acid probe set according to any one of claims 1 to 3, wherein the sum of X, Y and Z is 6 or smaller, wherein:

X is a distance in bases between the nucleotide (a) and a nucleotide $\beta$, the nucleotide $\beta$ exists in the fluorescent probe (A) and forms a base pair with a nucleotide $\alpha$, wherein the nucleotide $\alpha$ exists in the fluorescent probe binding region(b1) and is closest to the target nucleic acid binding region (b2);
Y is a distance in bases between the nucleotide $\alpha$ and a nucleotide $\gamma$, wherein the nucleotide $\gamma$ exists in the

target nucleic acid binding region (b2) and is closest to the nucleotide α; and Z is a distance in bases between a nucleotide δ and a nucleotide ε, wherein the nucleotide δ exists in the target nucleic acid (C) and forms a base pair with the nucleotide γ, and a nucleotide ε which exists in the nucleic acid including the the target nucleic acid (C) and has said guanine base.

**8.** A method for detecting a target nucleic acid, which comprises the following steps (1) to (4):

(1) hybridizing the nucleic acid probe set according to any one of claims 1 to 3 and the target nucleic acid (C) with each other,
(2) then measuring the fluorescence intensity of a hybridized complex of the nucleic acid probe set and target nucleic acid (C),
(3) conducting the steps (1) and (2) by changing a ratio of the nucleic acid probe set to the target nucleic acid, and
(4) comparing the fluorescence intensities obtained from the steps (2) and (3).

**9.** The method according to claim 8, wherein the sum of X, Y and Z is as defined in claim 7.

**10.** A real-time PCR method, which comprises using the nucleic acid probe set according to claim 1 to 3, wherein the sum of X, Y and Z is preferably as defined in claim 7.

**11.** A method for analyzing a nucleic acid for a base sequence polymorphism, which comprises the following steps (1) to (4) :

(1) hybridizing the nucleic acid probe set according to claim 1 to 3 and a target nucleic acid (C) with each other,
(2) then measuring a temperature dependence of fluorescence intensity with respect to a hybridized complex of the nucleic acid probe set and target nucleic acid (C),
(3) conducting the steps (1) and (2) by using another nucleic acid in place of the target nucleic acid, and
(4) comparing the temperature dependences of fluorescence intensity as obtained from the steps (2) and (3), wherein the sum of X, Y and Z is preferably as defined in claim 7.

**12.** A method, which comprises conducting a melting curve analysis on a complex of the nucleic acid probe set according to claim 1 to 3 and a target nucleic acid (C), wherein the sum of X, Y and Z is preferably as defined in claim 7.

**13.** The nucleic acid probe set according to claim 3, wherein the fluorescent probe (A) consisting of two fluorescent probes, which have different base sequences and are labeled with different fluorescent substances, respectively, and the binding probe (B) has two fluorescent probe binding regions having different base sequences.

**14.** An ABC-PCR method, which comprises using the nucleic acid probe set according to claim 13, wherein the sum of X, Y and Z is preferably as defined in claim 7.

## Patentansprüche

**1.** Satz von Nucleinsäuresonden, umfassend:

(A) eine fluoreszierende Sonde, gebildet aus einem Oligonucleotid mit (a) einem Nucleotid, das mit (d) einer fluoreszierenden Substanz markiert ist, wobei das Nucleotid (a) ein 5'-terminales Nucleotid des Oligonucleotids ist, und
(B) eine Bindesonde, gebildet aus einem Oligonucleotid mit (b1) einer Binderegion für eine fluoreszierende Sonde, an die die fluoreszierende Sonde (A) hybridiesieren kann, und (b2) einer Binderegion für eine Zielnucleinsäure, die mit einer Zielnucleinsäure (C) hybridisieren kann,

wobei der Satz an Nucleinsäuresonden so gestaltet ist, dass, wenn die fluoreszierende Sonde (A) an die Binderegion (b1) für die fluoreszierende Sonde hybridisiert und die Zielnucleinsäure (C) an die Binderegion (b2) für die Zielnucleinsäure hybridisiert, Fluoreszenz der fluoreszierenden Substanz durch Guanin in einer Nucleinsäure wie der Zielnucleinsäure (C) gelöscht wird, worin das Guanin und die fluoreszierende Substanz (d), mit der das Nucleotid (a) markiert ist, miteinander wechselwirken, um eine Fluoreszenzeigenschaft der fluoreszierenden Substanz (d) zu verändern, worin die Binderegion (b1) der Bindesonde (B) für die fluoreszierende Sonde sich am 5'-Ende der Bindesonde (B) befindet.

**2.** Satz von Nucleinsäuresonden, umfassend:

(A) eine fluoreszierende Sonde, gebildet aus einem Oligonucleotid mit (a) einem Nucleotid, das mit (d) einer fluoreszierenden Substanz markiert ist, wobei das Nucleotid (a) ein 3'-terminales Nucleotid des Oligonucleotids ist, und
(B) eine Bindesonde, gebildet aus einem Oligonucleotid mit (b1) einer Binderegion für eine fluoreszierende Sonde, an die die fluoreszierende Sonde (A) hybridiesieren kann, und (b2) einer Binderegion für eine Zielnucleinsäure, die mit einer Zielnucleinsäure (C) hybridisieren kann,

wobei der Satz an Nucleinsäuresonden so gestaltet ist, dass, wenn die fluoreszierende Sonde (A) an die Binderegion (b1) für die fluoreszierende Sonde hybridisiert und die Zielnucleinsäufe (C) an die Binderegion (b2) für die Zielnucleinsäure hybridisiert, Fluoreszenz der fluoreszierenden Substanz durch Guanin in einer Nucleinsäure wie der Zielnucleinsäure (C) gelöscht wird, worin das Guanin und die fluoreszierende Substanz (d), mit der das Nucleotid (a) markiert ist, miteinander wechselwirken, um eine Fluoreszenzeigenschaft der fluoreszierenden Substanz (d) zu verändern, worin die Binderegion (b1) der Bindesonde (B) für die fluoreszierende Sonde sich am 3'-Ende der Bindesonde (B) befindet.

**3.** Satz von Nucleinsäuresonden, umfassend:

(A) zwei fluoreszierende Sonden, von denen jede aus einem Oligonucleotid mit (a) einem Nucleotid, das mit (d) einer fluoreszierenden Substanz markiert ist, gebildet ist, worin das Nucleotid (a) einer ersten fluoreszierenden Sonde ein 5'-terminales Nucleotid eines Oligonucleotids der ersten fluoreszierenden Sonde ist, und das Nucleotid (a) einer zweiten fluoreszierenden Sonde ein 3'-terminales Nucleotid eines Oligonucleotids der zweiten fluoreszierenden Sonde ist, und
(B) eine Bindesonde aus einem Oligonucleotid mit (b1) zwei Binderegionen für fluoreszierende Sonden, die unterschiedliche Basensequenzen auf der Seite eines 5'-Endes und auf der Seite eines 3'-Endes des Oligonucleotids aufweisen und an die die fluoreszierenden Sonden (A) hybridisieren können, und (b2) eine Binderegion für eine Zielnucleinsäure, die mit einer Zielnucleinsäure (C) hybridisieren kann,

wobei der Satz an Nucleinsäuresonden so gestaltet ist, dass, wenn die fluoreszierende Sonde (A) an die Binderegion (b1) für die fluoreszierende Sonde hybridisiert und die Zielnucleinsäure (C) an die Binderegion (b2) für die Zielnucleinsäure hybridisiert, Fluoreszenz der fluoreszierenden Substanz durch Guanin in einer Nucleinsäure wie der Zielnucleinsäure (C) gelöscht wird, worin das Guanin und die fluoreszierende Substanz (d), mit der das Nucleotid (a) markiert ist, miteinander wechselwirken, um eine Fluoreszenzeigenschaft der fluoreszierenden Substanz (d) zu verändern.

**4.** Der Satz von Nucleinsäuresonden gemäß einem der Ansprüche 1 bis 3, worin die fluoreszierende Substanz (d) irgendeine ist ausgewählt aus der Gruppe bestehend aus Fluorescin, Ffuorescin-4-isothiocyanat, Tetrachlorfluorecin, Hexachlorfluorescin, Tetrabromsulfonfluorescin, EDANS, 6-JOE, Alexa Fluor 488, Alexa Fluor 532, Pacific Blue, Rhodamin 6G. Carboxyrhodamin 6G, Tetramethylrhodamin, Carboxytetramethylrhodamin und BODIPY-FL.

**5.** Der Satz an Nucleinsäuresonden gemäß einem der Ansprüche 1 bis 3, worin die eine oder zwei fluoreszierenden Sonden oder Sonden (A) jeweils 5 bis 50 Basen lang sind.

**6.** Der Satz an Nucleinsäuresonden gemäß einem der Ansprüche 1 bis 3, worin die Binderegion (b2) für die Zielnucleinsäure eine Länge von 5 bis 60 Basen aufweist.

**7.** Der Satz an Nucleinsäuresonden gemäß einem der Ansprüche 1 bis 3, worin die Summe von X, Y und Z 6 oder kleiner ist, worin:

X ein Abstand in Basen zwischen dem Nucleotid (a) und einem Nucleotid $\beta$ ist,
wobei Nucleotid $\beta$ in der fluoreszierenden Sonde (A) vorliegt und ein Basenpaar mit einem Nucleotid $\alpha$ bildet, worin das Nucleotid $\alpha$ in der Binderegion (b1) für die fluoreszierenden Sonde vorliegt und am nächsten zur Binderegion (b2) für die Zielnucleinsäure liegt;
Y ein Abstand in Basen zwischen dem Nucleotid $\alpha$ und einem Nucleotid $\gamma$ ist, worin das Nucleotid $\gamma$ in der Binderegion (b2) für die Zielnucleinsäure vorliegt und dem Nucleotid $\alpha$ am nächsten liegt; und
Z ein Abstand in Basen zwischen einem Nucleotid $\delta$ und einem Nucleotid $\varepsilon$ ist, worin das Nucleotid $\delta$ in der Zielnucleinsäure (C) vorliegt und ein Basenpaar mit dem Nucleotid $\gamma$ bildet, und ein Nucleotid $\varepsilon$ in der Nuclein-

säure wie der Zielnucleinsäure (C) vorliegt und die Guaninbase aufweist.

**8.** Verfahren zur Detektion einer Zielnucleinsäure, das die folgenden Schritte 1 bis 4 umfasst:

(1) Hybridiesieren des Satzes an Nucleinsäuresonden gemäß einem der Ansprüche 1 bis 3 mit der Zielnucleinsäure (C),
(2) dann Messen der Fluoreszenzintensität eines hybridisierten Komplexes des Satzes an Nucleinsäuresonden und der Zielnucleinsäure (C),
(3) Durchführen der Schritte 1 und 2 durch Ändern eines Verhältnisses des Satzes an Nucleinsäuresonden zu der Zielnucleinsäure und
(4) Vergleichen der Fluoreszenzintensitäten, die in den Schritten (2) und (3) erhalten wurden.

**9.** Das Verfahren gemäß Anspruch 8, worin die Summe von X, Y und Z wie in Anspruch 7 definiert ist.

**10.** Echtzeit-PCR-Verfahren, umfassend Verwenden des Satzes an Nucleinsäuresonden gemäß, einem der Ansprüche 1 bis 3, worin die Summe aus X, Y und Z vorzugsweise wie in Anspruch 7 definiert ist.

**11.** Verfahren zur Analyse einer Nucleinsäure auf einen Basensequenz-Polymorphismus, umfassend die folgenden Schritte (1) bis (4):

(1) Hybridisieren des Satzes an Nucleinsäuresonden gemäß einem der Ansprüche 1 bis 3 mit einer Zielnucleinsäure (C),
(2) dann Messen einer Temperaturabhängigkeit der Fluoreszenzintensität bezüglich eines hybridisierten Komplexes aus dem Satz an Nucleinsäuresonden und Zielnucleinsäure (C),
(3) Durchführen der Schritte 1 und 2 unter Verwendung einer anderen Nucleinsäure anstatt der Zielnucleinsäure, und
(4) Vergleichen der Temperaturabhängigkeit der Fluoreszenzintensität, wie in den Schritten (2) und (3) erhalten, worin die Summe aus X, Y und Z vorzugsweise wie in Anspruch 7 definiert ist.

**12.** Verfahren, umfassend Durchführen einer Schmelzkurvenanalyse eines Komplexes aus dem Satz an Nucleinsäuresonden gemäß einem der Ansprüche 1 bis 3 und einer Zielnucleinsäure (C), worin die Summe aus X, Y und Z vorzugsweise wie in Anspruch 7 definiert ist.

**13.** Der Satz von Nucleinsäuresonden gemäß Anspruch 3, worin die fluoreszierende Sonde (A) aus zwei Nucleinsäuresonden besteht, die verschiedene Basensequenzen aufweisen und mit verschiedenen fluoreszierenden Substanzen markiert sind, und die Bindesonde (B) zwei Binderegionen für fluoreszierende Sonden aufweist, die verschiedene Basensequenzen aufweisen.

**14.** ABC-PCR-Verfahren, umfassend Verwenden des Satzes an Nucleinsäuresonden gemäß Anspruch 13, worin die Summe aus X, Y und Z vorzugsweise wie in Anspruch 7 definiert ist.

## Revendications

**1.** Jeu de sondes d'acide nucléique comprenant :

(A) une sonde fluorescente qui est formée d'un oligonucléotide contenant (a) un nucléotide marqué avec (d) une substance fluorescente, le nucléotide (a) étant un nucléotide 5'-terminal dudit oligonucléotide, et
(B) une sonde de liaison formée d'un oligonucléotide comportant (b1) une région se liant à une sonde fluorescente, à laquelle la sonde fluorescente (A) peut s'hybrider, et (b2) une région se liant à un acide nucléique cible qui peut s'hybrider à un acide nucléique cible (C),

lequel jeu de sondes d'acide nucléique est conçu de sorte que, lorsque la sonde fluorescente (A) s'hybride à la région se liant à une sonde fluorescente (b1) et l'acide nucléique cible (C) s'hybride à la région se liant à un acide nucléique cible (b2), la fluorescence provenant de la substance fluorescente est éteinte par la guanine dans un acide nucléique qui contient l'acide nucléique cible (C), où ladite guanine et la substance fluorescente (d) marquée sur le nucléotide (a) interagissent l'une avec l'autre pour changer le caractère fluorescent de la substance fluorescente (d), et où ladite région se liant à une sonde fluorescente (b1) de la sonde de liaison (B) est située sur l'extrémité 5' de la sonde de liaison (B).

**2.** Jeu de sondes d'acide nucléique comprenant :

(A) une sonde fluorescente qui est formée d'un oligonucléotide contenant (a) un nucléotide marqué avec (d) une substance fluorescente, le nucléotide (a) étant un nucléotide $3^1$-terminal dudit oligonucléotide, et
(B) une sonde de liaison formée d'un oligonucléotide comportant (b1) une région se liant à une sonde fluorescente, à laquelle la sonde fluorescente (A) peut s'hybrider, et (b2) une région se liant à un acide nucléique cible qui peut s'hybrider à un acide nucléique cible (C),

lequel jeu de sondes d'acide nucléique est conçu de sorte que, lorsque la sonde fluorescente (A) s'hybride à la région se liant à une sonde fluorescente (b1) et l'acide nucléique cible (C) s'hybride à la région se liant à un acide nucléique cible (b2), la fluorescence provenant de la substance fluorescente est éteinte par la guanine dans un acide nucléique qui contient l'acide nucléique cible (C), où ladite guanine et la substance fluorescente (d) marquée sur le nucléotide (a) interagissent l'une avec l'autre pour changer le caractère fluorescent de la substance fluorescente (d), et où ladite région se liant à une sonde fluorescente (b1) de la sonde de liaison (B) est située sur l'extrémité 3' de la sonde de liaison (B).

**3.** Jeu de sondes d'acide nucléique comprenant :

(A) deux sondes fluorescentes, chacune étant formée d'un oligonucléotide contenant (a) un nucléotide marqué avec (d) une substance fluorescente, le nucléotide (a) de la première sonde fluorescente étant un nucléotide 5'-terminal d'un oligonucléotide de la première sonde fluorescente, et le nucléotide (a) de la deuxième sonde fluorescente étant un nucléotide 3'-terminal d'un oligonucléotide de la deuxième sonde fluorescente, et
(B) une sonde de liaison formée d'un oligonucléotide comportant (b1) deux régions se liant à une sonde fluorescente présentant des séquences de bases différentes du côté de l'extrémité 5' et du côté de l'extrémité 3' dudit oligonucléotide et auxquelles les sondes fluorescentes (A) peuvent s'hybrider, et (b2) une région se liant à un acide nucléique cible qui peut s'hybrider à un acide nucléique cible (C),

lequel jeu de sondes d'acide nucléique est conçu de sorte que, lorsque les sondes fluorescentes (A) s'hybrident aux régions se liant à une sonde fluorescente (b1) et l'acide nucléique cible (C) s'hybride à la région se liant à un acide nucléique cible (b2), la fluorescence provenant de la substance fluorescente est éteinte par la guanine dans un acide nucléique qui contient l'acide nucléique cible (C), si bien que guanine et la substance fluorescente (d) marquée sur le nucléotide (a) interagissent l'une avec l'autre pour changer le caractère fluorescent de la substance fluorescente (d).

**4.** Jeu de sondes d'acide nucléique selon l'une quelconque des revendications 1 à 3, dans lequel la substance fluorescente (d) est l'une quelconque choisie dans l'ensemble constitué par la fluorescéine, le 4-isothiocyanate de fluorescéine, la tétrachlorofluorescéine, l'hexachlorofluorescéine, la tétrabromosulfonefluorescéine, EDANS, 6-JOE, Alexa Fluor 488, Alexa Fluor 532, Pacific Blue, la rhodamine 6G, la carboxyrhodamine 6G, la tétraméthylrhodamine, la carboxytétraméthylrhodamine et BODIPY-FL.

**5.** Jeu de sondes d'acide nucléique selon l'une quelconque des revendications 1 à 3, dans lequel l'une ou les deux des sondes (A) présente(nt) chacune une longueur de 5 à 50 bases.

**6.** Jeu de sondes d'acide nucléique selon l'une quelconque des revendications 1 à 3, dans lequel la région se liant à un acide nucléique cible (b2) présente une longueur de 5 à 60 bases.

**7.** Jeu de sondes d'acide nucléique selon l'une quelconque des revendications 1 à 3, dans lequel la somme de X, Y et Z vaut 6 ou moins, où :

X est la distance en bases entre le nucléotide (a) et un nucléotide $\beta$, le nucléotide $\beta$ existant dans la sonde fluorescente (A) et formant une paire de bases avec un nucléotide $\alpha$, le nucléotide $\alpha$ existant dans la région se liant à une sonde fluorescente (b1) et étant le plus proche de la région se liant à un acide nucléique cible (b2) ;
Y est la distance en bases entre le nucléotide $\alpha$ et un nucléotide $\gamma$, le nucléotide $\gamma$ existant dans la région se liant à un acide nucléique cible (b2) et étant le plus proche du nucléotide $\alpha$ ; et
Z est la distance en bases entre un nucléotide $\delta$ et un nucléotide $\varepsilon$, le nucléotide $\delta$ existant dans l'acide nucléique cible (C) et formant une paire de bases avec le nucléotide $\gamma$, et le nucléotide $\varepsilon$ existant dans l'acide nucléique contenant l'acide nucléique cible (C) et comportant ladite base de guanine.

**8.** Procédé pour détecter un acide nucléique cible, qui comprend les étapes (1) à (4) suivantes :

(1) hybridation du jeu de sondes d'acide nucléique selon l'une quelconque des revendications 1 à 3 et de l'acide nucléique cible (C) l'un avec l'autre,
(2) puis mesure de l'intensité de fluorescence d'un complexe hybridé du jeu de sondes d'acide nucléique et de l'acide nucléique cible (C),
(3) mise en oeuvre des étapes (1) et (2) avec un changement du rapport du jeu de sondes d'acides nucléiques sur l'acide nucléique cible, et
(4) comparaison des intensités de fluorescence obtenues dans les étapes (2) et (3).

**9.** Procédé selon la revendication 8, dans lequel la somme de X, Y et Z est telle que définie dans la revendication 7.

**10.** Procédé PCR en temps réel, qui comprend l'utilisation du jeu de sondes d'acide nucléique selon les revendications 1 à 3, dans lequel la somme de X, Y et Z est de préférence telle que définie dans la revendication 7.

**11.** Procédé pour analyser un acide nucléique pour un polymorphisme de séquences de bases, qui comprend les étapes (1) à (4) suivantes :

(1) hybridation du jeu de sondes d'acide nucléique selon les revendications 1 à 3 et d'un acide nucléique cible (C) l'un avec l'autre,
(2) puis mesure d'une dépendance à la température de l'intensité de fluorescence en regard d'un complexe hybridé du jeu de sondes d'acide nucléique et de l'acide nucléique cible (C),
(3) mise en oeuvre des étapes (1) et (2) par utilisation d'un autre acide nucléique à la place de l'acide nucléique cible, et
(4) comparaison des dépendances à la température des intensités de fluorescence telles qu'obtenues dans les étapes (2) et (3),

dans lequel la somme de X, Y et Z est de préférence telle que définie dans la revendication 7.

**12.** Procédé qui comprend la mise en oeuvre d'une analyse de courbe de fusion sur un complexe du jeu de sondes d'acide nucléique selon les revendications 1 à 3 et d'un acide nucléique cible (C), dans lequel la somme de X, Y et Z est de préférence telle que définie dans la revendication 7.

**13.** Jeu de sondes d'acide nucléique selon la revendication 3, dans lequel la sonde fluorescente (A) est constituée de deux sondes fluorescentes, qui présentent des séquences de bases différentes et sont marquées avec des substances fluorescentes différentes, respectivement, et la sonde de liaison (B) comporte deux régions se liant à une sonde fluorescente ayant des séquences de bases différentes.

**14.** Procédé ABC-PCR, qui comprend l'utilisation du jeu de sondes d'acide nucléique selon la revendication 13, dans lequel la somme de X, Y et Z est de préférence telle que définie dans la revendication 7.

Fig. 1

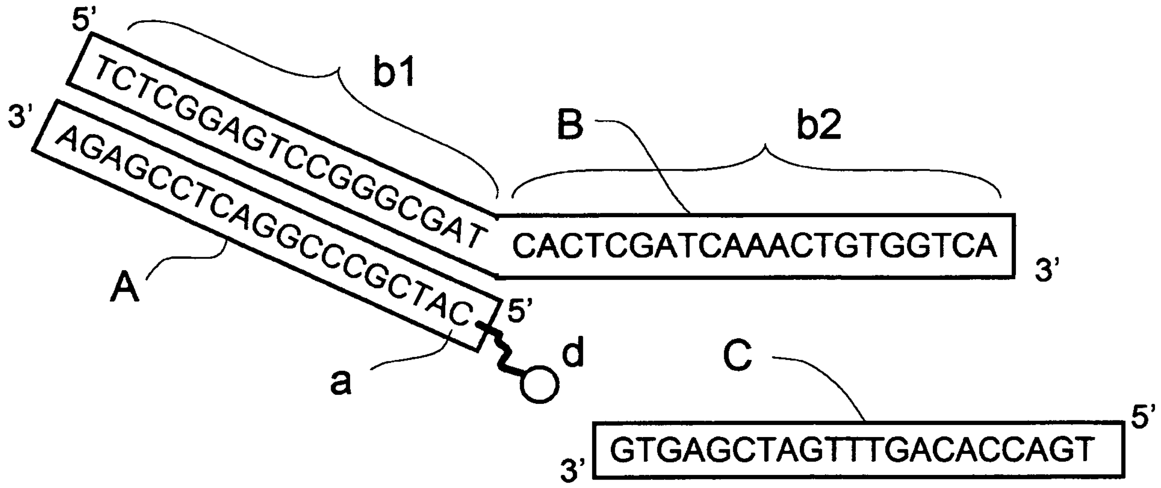
5'
3'
TCTCGGAGTCCGGGCGAT
AGAGCCTCAGGCCCGCTAC
b1
B
b2
CACTCGATCAAACTGTGGTCA
3'
A
5'
a
d
C
GTGAGCTAGTTTGACACCAGT
5'
3'

Fig. 2

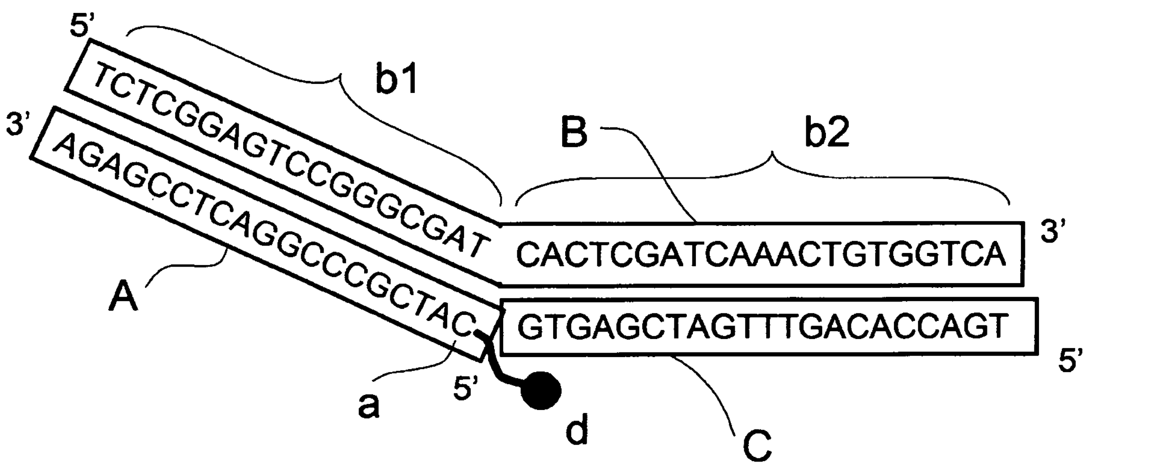
5'
3'
TCTCGGAGTCCGGGCGAT
AGAGCCTCAGGCCCGCTAC
b1
B
b2
CACTCGATCAAACTGTGGTCA
3'
A
GTGAGCTAGTTTGACACCAGT
5'
a
5'
d
C

Fig. 3

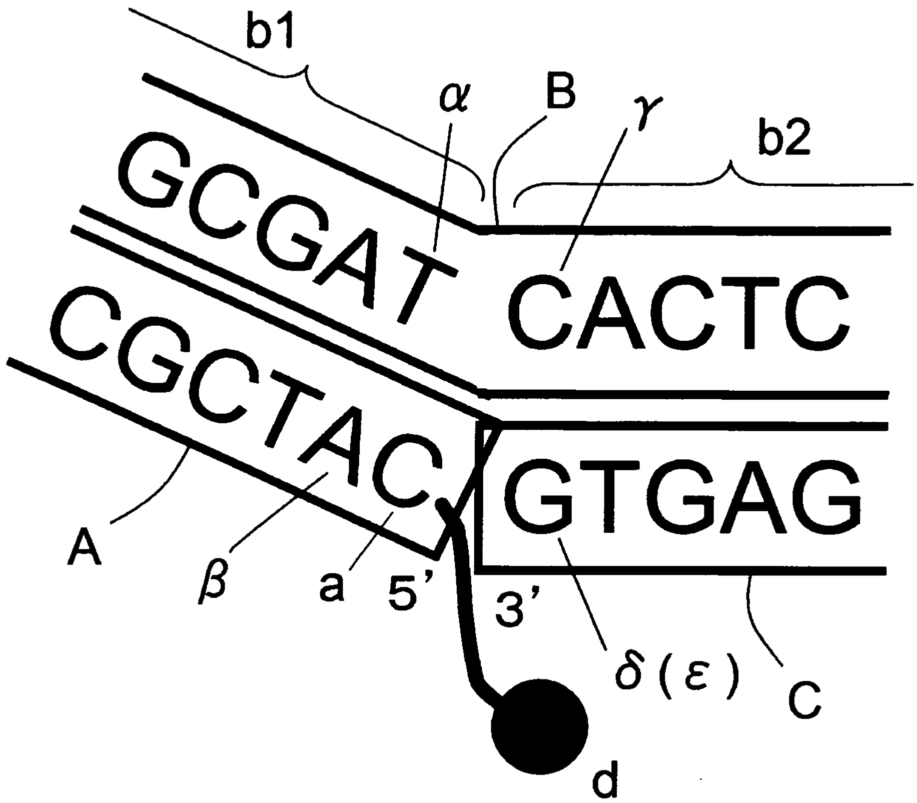

Fig. 4

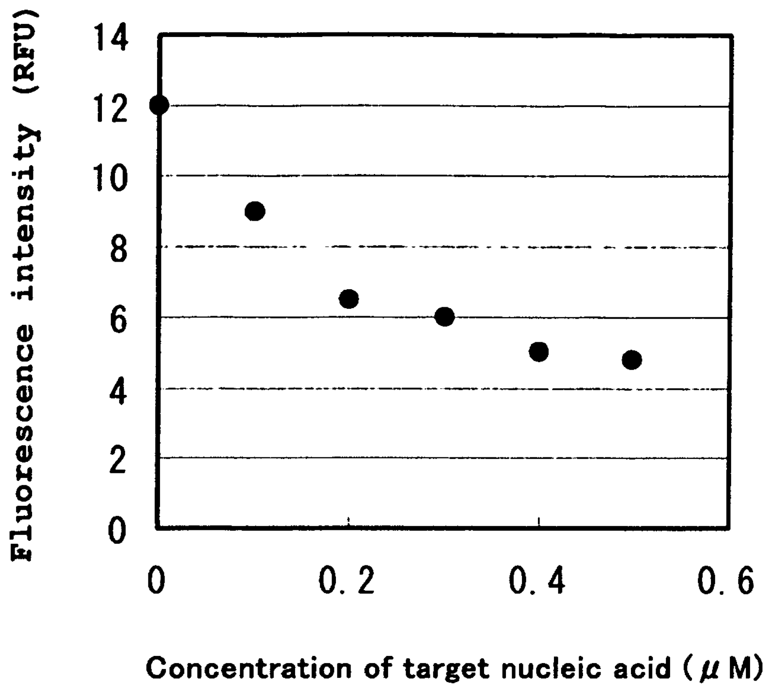
Fluorescence intensity (RFU)
14
12
10
8
6
4
2
0
0
0. 2
0. 4
0. 6
Concentration of target nucleic acid (μM)

Fig. 5

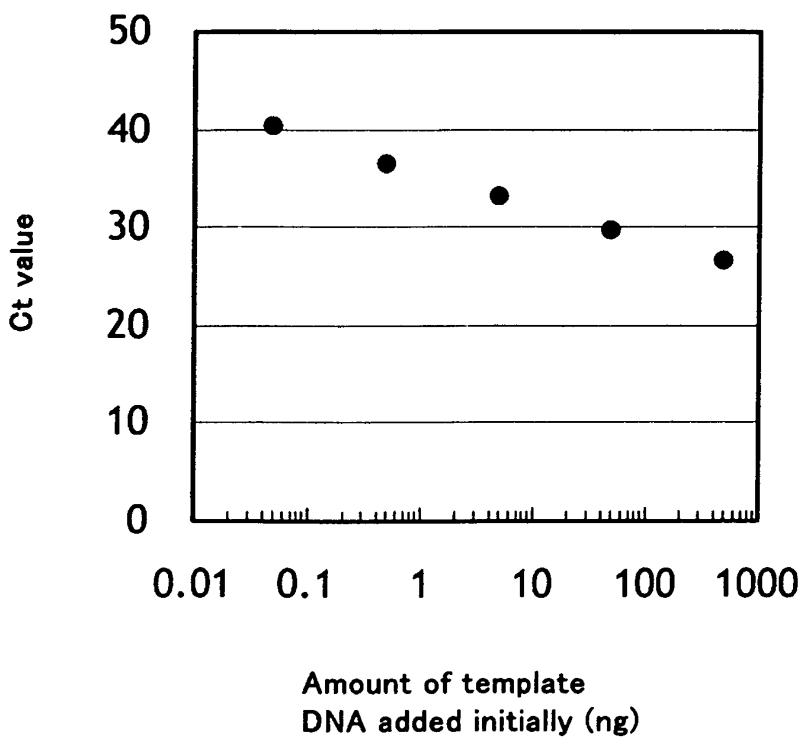
Ct value
50
40
30
20
10
0
0.01
0.1
1
10
100
1000
Amount of template
DNA added initially (ng)

Fig. 6

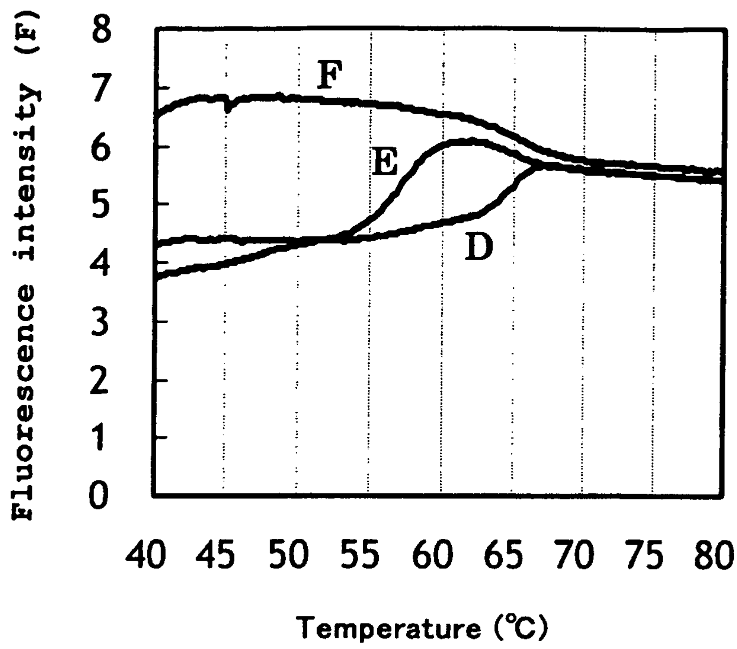
Fluorescence intensity (F)
8
7
6
5
4
3
2
1
0
F
E
D
40 45 50 55 60 65 70 75 80
Temperature (℃)

Fig. 7

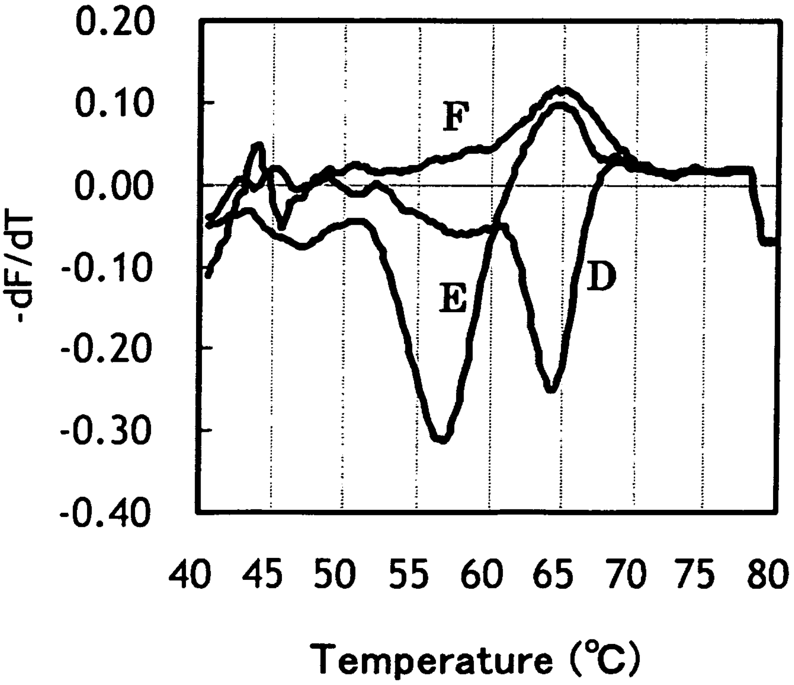
-dF/dT
0.20
0.10
0.00
-0.10
-0.20
-0.30
-0.40
F
E
D
40 45 50 55 60 65 70 75 80
Temperature (℃)

Fig. 8

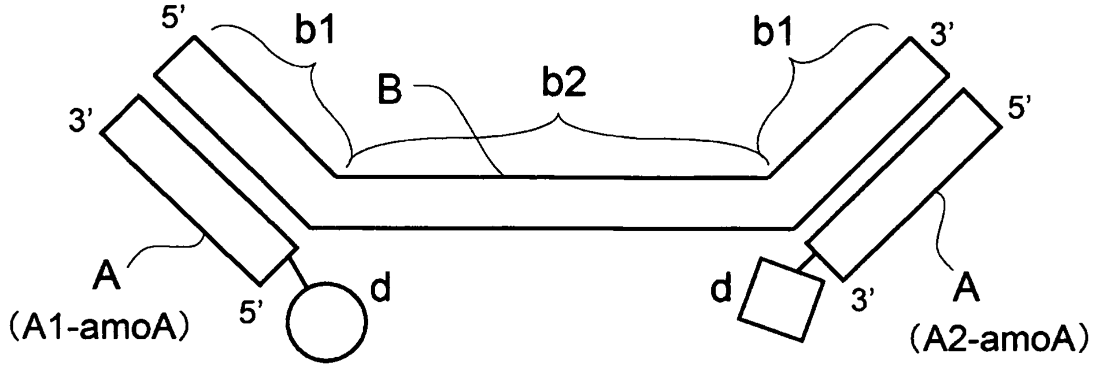
5'
b1
b1
3'
B
b2
3'
5'
A
(A1-amoA)
5'
d
d
3'
A
(A2-amoA)

Fig. 9

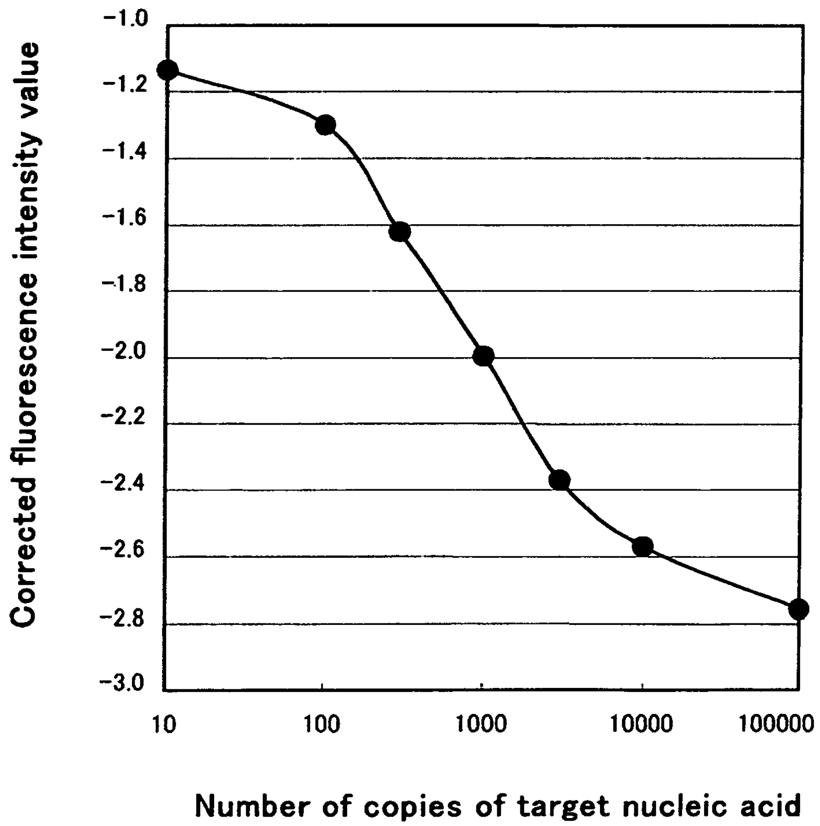
Corrected fluorescence intensity value
-1.0
-1.2
-1.4
-1.6
-1.8
-2.0
-2.2
-2.4
-2.6
-2.8
-3.0
10
100
1000
10000
100000
Number of copies of target nucleic acid

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- JP 3437816 B
- JP 2002119291 A


### Non-patent literature cited in the description


- *Nature Biotechnology,* 1996, vol. 14, 303-308
- *Applied and Environmental Microbiology,* 1997, vol. 63, 1143-1147
- *Nucleic Acids Research,* 1996, vol. 24, 4532-4535
- *ANALYTICAL BIOCHEMISTRY,* 1998, vol. 225, 32-38
- **TANI et al.** *Analytical Chemistry*
- **HIGUCHI et al.** *NucleicAcids Res.,* 1988, vol. 16, 7351-5367